# EUROPEAN PATENT SPECIFICATION

(11) **EP 2 938 622 B1**
(45) Date of publication and mention of the grant of the patent: **10.08.2016**
(21) Application number: 13721103.3
(22) Date of filing: 25.03.2013
(51) Int. Cl.: C07F 15/00, B01J 31/22

(54) **NOVEL RUTHENIUM COMPLEXES, A METHOD OF PRODUCING THEM, AND THEIR USE IN OLEFIN METATHESIS**
NEUARTIGE RUTHENIUMKOMPLEXE, VERFAHREN ZUR HERSTELLUNG DAVON UND DEREN VERWENDUNG IN DER OLEFINMETATHESE
NOUVEAUX COMPLEXES DE RUTHÉNIUM, LEUR PROCÉDÉ DE PRODUCTION ET UTILISATION DANS LA MÉTATHÈSE D'OLÉFINES

(30) Priority: 28.12.2012 PL 40229312
(43) Date of publication of application: 04.11.2015
(73) Proprietor: Instytut Chemii Organicznej Pan, 01-244 Warszawa (PL)
(72) Inventor: GRELA, Karol, 01-471 Warszawa (PL); GAWIN, Rafal, 02-759 Warszawa (PL); PIECZYKOLAN, Michal, 22-400 Zamosc (PL)
(74) Representative: Witek, Rafal
(86) International application number: PCT/IB2013/052368
(87) International publication number: WO 2014/102622

(56) References cited:
- GEORGIOS C. VOUGIOUKALAKIS ET AL: "Ruthenium-Based Heterocyclic Carbene-Coordinated Olefin Metathesis Catalysts +", CHEMICAL REVIEWS, vol. 110, no. 3, 10 March 2010 (2010-03-10), pages 1746-1787, XP055071994, ISSN: 0009-2665, DOI: 10.1021/cr9002424 cited in the application
- MARK S. KERR ET AL: "An Efficient Synthesis of Achiral and Chiral 1,2,4-Triazolium Salts: Bench Stable Precursors for N - Heterocyclic Carbenes", THE JOURNAL OF ORGANIC CHEMISTRY, vol. 70, no. 14, 1 July 2005 (2005-07-01), pages 5725-5728, XP055072016, ISSN: 0022-3263, DOI: 10.1021/jo050645n
- RAFAL GAWIN ET AL: "Testing New Ruthenium Complexes bearing Chiral 1,2,4-Triazol-5-ylidene -Ligands as Catalysts for Asymmetric Olefin Metathesis", SYNLETT, vol. 24, no. 10, 5 June 2013 (2013-06-05), pages 1250-1254, XP055072011, ISSN: 0936-5214, DOI: 10.1055/s-0033-1338877

## Description

The present invention relates to novel chiral, non-racemic complexes of ruthenium, for use as (pre)catalysts in the olefin metathesis reactions, a method of producing them and their use in the metathesis reactions of olefins (Chem. Rev. 2010, 110, 1746-1787).

The state of the art encompasses chiral, non-racemic carbene ruthenium complexes that act as (pre)catalysts, which make it possible to perform asymmetric metathesis reactions. These complexes posses NHC carbenes as a ligand that are that are derivatives of imidazolo-4,5-dihydro-2-ylidene (Chem. Soc. Rev. 2012, 41, 4389-4408). The synthesis of known chiral non-racemic ruthenium (pre)catalysts using metathesis reactions consists of many stages, and their precursors are difficult to obtain (J. Am. Chem. Soc. 2006, 128, 1840-1846; Organometallics 2007, 26, 2945-2949; J. Am. Chem. Soc. 2002, 124, 4954-4955).

Unexpectedly, it was shown that novel, chiral, non-racemic carbene ruthenium complexes defined by Formula **1,** 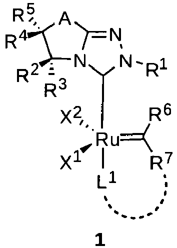 containing as a ligand chiral, non-racemic NHC carbenes that are derivatives of 1,2,4-triazol-5-ylidene make it possible to perform asymmetric metathesis reactions. The precursors of complex **1** are optically active amino-alcohols, which may be obtained from inexpensive amino-acids of natural origin. The synthesis of complex **1** consists of a lesser number of stages, in comparison to the syntheses of known chiral non-racemic ruthenium (pre)catalysts for metathesis reactions.
Complexes defined by Formula **1,** according to the present invention are useful in a wide range of asymmetric metathesis reactions. The subject of the present invention are novel, chiral non-racemic ruthenium complexes defined by the general Formula **1,** 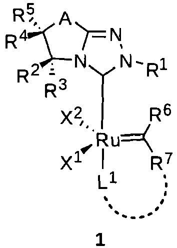 in which:
R¹ denotes a C₅-C₂₄ perfluoroaryl;
R², R³, R⁴ and R⁵ independently of one another denote a hydrogen atom, a halogen atom, a C₁-C₂₅ alkyl, a C₃-C₇ cycloalkyl, a C₁-C₂₅ alkoxyl, a C₅-C₂₄ aryloxyl, a C₅-C₂₀ heteroaryloxyl, a C₅-C₂₄ aryl, a C₅-C₂₀ heteroaryl, a C₇-C₂₄ aralkyl, a C₅-C₂₄ perfluoroaryl, or 3-12 membered heterocycle, wherein the groups R², R³, R⁴ and R⁵ may be mutually connected into a ring;
A denotes a -CH₂-, -O- or -OCH₂- group;
R⁶ and R⁷ independently of one another denote a hydrogen atom, a halogen atom, a C₁-C₂₅ alkyl, a C₁-C₂₅ perfluoroalkyl, a C₂-C₂₅ alkene, a C₃-C₇ cycloalkyl, a C₂-C₂₅ alkenyl, a C₃-C₂₅ cycloalkenyl, a C₂-C₂₅ alkynyl, a C₃-C₂₅ cycloalkynyl, a C₁-C₂₅ alkoxyl, a C₅-C₂₄ aryloxyl, a C₅-C₂₀ heteroaryloxyl, a C₅-C₂₄ aryl, a C₅-C₂₀ heteroaryl, a C₇-C₂₄ aralkyl, a C₅-C₂₄ perfluoroaryl, a 3-12 membered heterocycle wherein the alkyl groups may be mutually connected into a ring, wherein R⁶ and R⁷ preferably denote a hydrogen, an aryl substituted with a nitro (-NO₂), cyanide (-CN), carboxyl (-COOH), ester (-COOR'), amide (-CONR'₂), sulfonyl (-SO₂R'), formyl (-CHO), sulfonamide (-SO₂NR'₂), or ketone (-COR') group, in which R' has the following meaning: a C₁-C₅ alkyl, a C₁-C₅ perfluoroalkyl, a C₅-C₂₄ aryl, a C₇-C₂₄ aralkyl, a C₅-C₂₄ perfluoroaryl;
L¹ denotes a neutral ligand;
X¹ and X² denote an anionic ligand.

The neutral ligand L¹ is selected from among the groups encompassing P(R')₃, P(OR')₃, O(R')₂, N(R')₃, where each R' independently denotes a C₁-C₁₂ alkyl, a C₃-C₁₂ cycloalkyl, a C₅-C₂₀ aryl, a C₇-C₂₄ aralkyl, a C₅-C₂₄ perfluoroaryl, or a 5-12 membered heteroaryl; The neutral ligand L¹ may also be a pyridine or substituted pyridine;

The anionic ligands X¹ and X² are independently selected from groups encompassing halide anions, as well as -CN, -SCN, -OR', -SR', -O(C=O)R', -O(SO₂)R', -OSi(R')₃ groups, where R' denotes a C₁-C₁₂ alkyl, a C₃-C₁₂ cycloalkyl, a C₂-C₁₂ alkenyl, or a C₅-C₂₀ aryl, which may possibly be substituted with at least one C₁-C₁₂ alkyl, a C₁-C₁₂ perfluoroalkyl, a C₁-C₁₂ alkoxyl, a C₅-C₂₄ aryloxyl, a C₅-C₂₀ heteroaryloxyl or a halogen atom.

In a preferable embodiment of the present invention, complex **1** has a structure defined by the general Formula **1a** 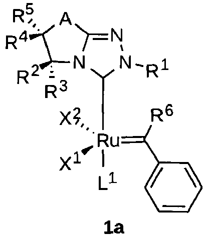 in which:
R¹, R², R³, R⁴, R⁵, A, L¹, X¹, and X² have the same meaning as in Formula **1**
R⁶ denotes a hydrogen.

In another preferable embodiment, complex **1** has a structure defined by the general Formula **1b** 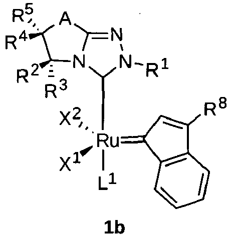 in which:
R¹ R², R³, R⁴, R⁵, A, L¹, X¹ and X² have the same meaning as in Formula **1**
R⁸ denotes a hydrogen atom, a C₅-C₂₀ aryl, a C₅-C₂₀ heteroaryl, a C₇-C₂₄ aralkyl, vinyl or allenyl.

In another preferable embodiment, complex **1** has a structure defined by the general Formula **1c** 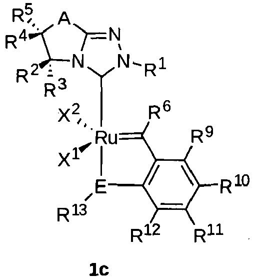 in which:
R¹, R², R³, R⁴, R⁵, A, L¹, X¹ and X² have the same meaning as in Formula 1
R⁶ denotes a hydrogen;
R⁹, R¹⁰, R¹¹, R¹² independently of one another denote a hydrogen atom, a halogen atom, a C₁-C₂₅ alkyl, a C₁-C₂₅ perfluoroalkyl, a C₂-C₂₅ alkene, a C₃-C₇ cycloalkyl, a C₂-C₂₅ alkenyl, a C₃-C₂₅ cycloalkenyl, a C₂-C₂₅ alkynyl, a C₃-C₂₅ cycloalkynyl, a C₅-C₂₄ aryl, a C₅-C₂₀ heteroaryl, a C₇-C₂₄ aralkyl, a C₅-C₂₄ perfluoroaryl, or 3-12 membered heterocycle wherein the alkyl groups may be mutually connected into a ring, an ether (-OR'), thioether (-SR'), nitro (-NO₂), cyanide (-CN), carboxyl (-COOH), ester (-COOR'), amide (-CONR'₂), imide (-CONR'COR'), amino (-NR'₂), amide (NR'COR'), sulfonamide (-NR'SO₂R'), sulfonyl (-SO₂R'), formyl (-CHO), sulfonamide (-SO₂NR'₂) or ketone (-COR') group, in which R' has the following meaning: a C₁-C₅ alkyl, a C₁-C₅ perfluoroalkyl, a C₅-C₂₄ aryl, a C₅-C₂₄ perfluoroaryl, a C₇-C₂₄ aralkyl, wherein the alkyl groups may be mutually connected into a ring; preferably, R⁹, R¹⁰, R¹¹, R¹² denotes a hydrogen;
R¹³ denotes a hydrogen atom, a C₁-C₂₅ alkyl, a C₁-C₂₅ perfluoroalkyl, a C₃-C₇ cycloalkyl, a C₅-C₂₄ aryl, a C₅-C₂₄ perfluoroaryl, a C₅-C₂₀ heteroaryl, a C₇-C₂₄ aralkyl, a 3-12 membered heterocycle wherein the alkyl groups may be mutually connected into a ring, an acyl -COR', cyanide (-CN), carboxyl (-COOH), ester (-COOR'), amide (-CONR'₂), sulfonyl (-SO₂R') formyl (-CHO), sulfonamide (-SO₂NR'₂), or ketone (-COR') group, in which R' has the following meaning: a C₁-C₅ alkyl, a C₁-C₅ perfluoroalkyl, a C₅-C₂₄ aryl, a C₅-C₂₄ perfluoroaryl, or a C₇-C₂₄ aralkyl;
E denotes an oxygen atom.

Preferably, a complex according to the present invention is characterised in that
R¹ denotes pentafluorophenyl;
R², R³, R⁴ and R⁵ independently of one another denote a hydrogen atom, a C₁-C₂₅ alkyl, a C₃-C₇ cycloalkyl, a C₅-C₂₄ aryl, a C₅-C₂₀ heteroaryl, a C₇-C₂₄ aralkyl, a C₅-C₂₄ perfluoroaryl, or a 3-12 membered heterocycle, wherein the groups R², R³, R⁴ and R⁵ may be mutually connected into a ring;
A denotes a -CH₂-, -O-, or -OCH₂- group;
L¹ denotes a neutral ligand selected from groups encompassing tricyclohexylphosphine, triphenylphosphine, pyridine, 3-bromopyridine;
X¹ and X² denote chlorine, bromine or iodine.

The subject of the present invention is also a method of producing complexes of ruthenium defined by the general Formula **1,** which encompasses reactions of carbene complexes of ruthenium defined by Formula **2,** 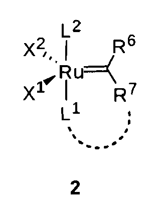 in which:
R⁶, R⁷, L¹, X¹ and X² have the same meaning as in Formula 1
L¹ and L² have the same meaning as L¹ in Formula **1**
with chiral non-racemic carbenes defined by the general Formula **3** or with chiral non-racemic complexes of silver defined by the general Formula **4** or with carbenes formed from precursors of chiral non-racemic carbenes defined by the general Formula **5** or **6,** 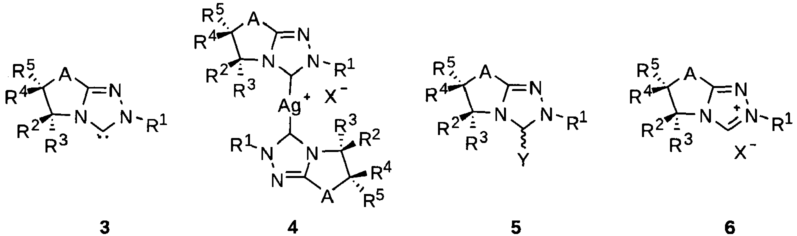 in which:
R¹, R², R³, R⁴, R⁵ and A have the same meaning as in Formula **1**
X denotes a halide anion or BF₄⁻, PF₆⁻, ClO₄⁻
Y denotes alkoxyl, pentafluorophenyl, -CCl₃.

In a preferable embodiment as a precursor of complex **1** use is made of complex 2 defined by the general Formula **2a** 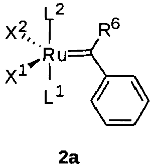 in which:
X¹ and X² have the same meaning as in Formula **1;**
L¹ and L² have the same meaning as L¹ in Formula **1;**
R⁶ denotes a hydrogen.

In another preferable embodiment as a precursor of complex **1** use is made of complex **2** defined by the general Formula **2b** 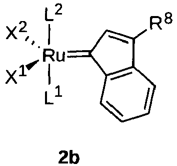 in which
X¹ and X² have the same meaning as in Formula **1;**
L¹ and L² have the same meaning as L¹ in Formula **1;**
R⁸ denotes a hydrogen atom, a C₅-C₂₀ aryl, a C₅-C₂₀ heteroaryl, a C₇-C₂₄ aralkyl, vinyl or allenyl.

In another preferable embodiment as a precursor of complex **1** use is made of complex 2 defined by the general Formula **2c** 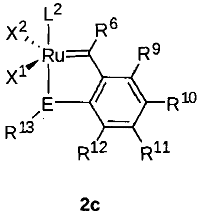 in which
X¹ and X² have the same meaning as in Formula **1;**
L² has the same meaning as L¹ in Formula **1;**
R⁶, R⁹, R¹⁰, R¹¹, R¹² and R¹³ have the same meaning as in Formula **1c;**
E has the same meaning as in Formula **1c.**

The production of complexes of ruthenium **1** from complexes of ruthenium **2** and carbenes **3** or with complexes of silver **4** or with precursor carbenes 5 is shown in Scheme I:

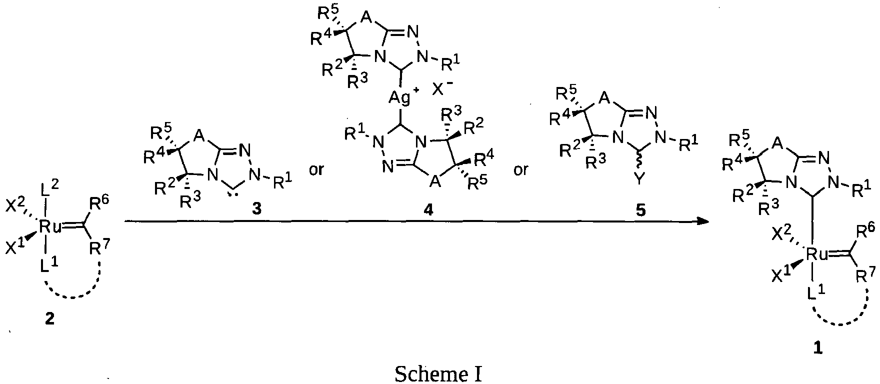

Preferably, reactions are conducted over a period from 1 minute to 250 hours at a temperature from 0°C to 150°C.

Preferably, the reactions are conducted in aromatic hydrocarbons, aliphatic hydrocarbons, ethers or mixtures thereof.

Preferably, the reaction is conducted in a solvent selected from among toluene, *n*-hexane, tetrahydrofuran, dioxane and diethyl ether.

The production of complexes of ruthenium **1** from complexes of ruthenium **2** and carbenes produced from precursor carbenes **6** is shown in Scheme II:

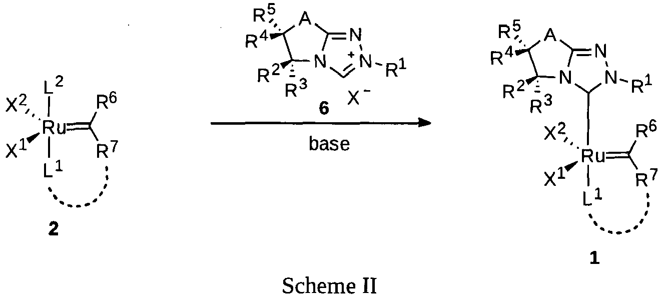

Preferably, the reaction is conducted over a period from 1 minute to 250 hours at a temperature from 0°C to 150°C.

Preferably, the reaction is conducted in a protic or aprotic solvent, a chlorinated solvent or in a aromatic hydrocarbon solvent, or in mixtures thereof.

Preferably, the reaction is conducted in a solvent selected from among tetrahydrofuran and/or toluene and/or methylene chloride.

Preferably, the reaction is conducted in the presence of organic or inorganic bases.

Preferably, the reaction is conducted in the presence of bases selected from among: potassium *tert-*butanolate, potassium *tert*-amylate, potassium N,N-bis(trimethylsilyl)amide, sodium hydride.

The subject of the present invention is also the use of complexes of ruthenium defined by Formula **1** as (pre)catalysts in metathesis reactions and cycloisomerisation of olefins.

Preferably, ruthenium complexes defined by Formula 1 are used as (pre)catalysts in asymmetric ring closing metathesis (ARCM), in asymmetric ring opening metathesis with subsequent cross metathesis (AROM/CM) as well as in asymmetric cross metathesis (ACM).

The term "halogen atom" denotes an element selected from among F, Cl, Br and I.

The term "halide anion" denotes a fluoride, chloride, bromide or iodide anion.

The term "carbene" denotes a molecule containing a neutral carbon atom with the valence number 2 and two unpaired valence electrons. The term "carbene" also encompasses carbene analogues in which the carbon atom has been substituted by another chemical element such as boron, silicon, germanium, tin, lead, nitrogen, phosphorus, sulphur, selenium and tellurium.

The term "alkyl" refers to a saturated, linear or branched hydrocarbon substituent with an indicated number of carbon atoms. Examples of alkyl substituents are -methyl, -ethyl, -*n*-propyl, -n-butyl, -*n*-pentyl, -*n*-hexyl, -*n*-heptyl, -*n*-octyl, -*n*-nonyl, and -*n*-decyl.
Representative branched -(C₁-C₁₀) alkyls encompass -isopropyl, -*sec*-butyl, -isobutyl, -*tert*-butyl, -isopentyl, -neopentyl, -1-methylbutyl, -2-methylbutyl, -3-methylbutyl, -1,1-dimethylpropyl, -1,2-dimethylpropyl, -1-methylpentyl, -2-methylpentyl, -3-methylpentyl, -4-methylpentyl, -1-ethylbutyl, -2-ethylbutyl, -3-ethylbutyl, -1,1-dimethylbutyl, -1,2-dimethylbutyl, -1,3-dimethylbutyl, -2,2-dimethylbutyl, -2,3-dimethylbutyl, -3,3-dimethylbutyl, -1-methylhexyl, -2-methylhexyl, -3-methylhexyl, -4-methylhexyl, -5-methylhexyl, -1,2-dimethylpentyl, -1,3- dimethylpentyl, -1,2-dimethylhexyl, -1,3-dimethylhexyl, -3,3-dimethylhexyl, -1,2-dimethylheptyl, -1,3-dimethylheptyl, and -3,3-dimethylheptyl and the like.

The term "perfluoroalkyl" denotes an alkyl group as defined above, wherein all hydrogen atoms have been substituted by identical or different halide atoms.

The term "cycloalkyl" refers to a saturated mono- or polycyclic hydrocarbon substituent with the indicated number of carbon atoms.
Examples cycloalkyl substituents include -cyclopropyl; -cyclobutyl, -cyclopentyl, -cyclohexyl, -cycloheptyl, -cyclooctyl, -cyclononyl, -cyclfromecyl, and the like.

The term "alkoxyl" refers to alkyl or cycloalkyl substituents as defined above and attached via an oxygen atom.

The term "alkenyl" refers to an unsaturated, linear, or branched acyclic hydrocarbon substituent with the indicated number of carbon atoms and containing at least one double carbon-carbon bond. Examples of an alkenyl substituent include -vinyl, -allyl, -1-butenyl, -2-butenyl, -isobutylenyl, -1-pentenyl, -2-pentenyl, -3-methyl-1-butenyl, -2-methyl-2-butenyl, -2,3-dimethyl-2-butenyl, -1-hexenyl, -2-hexenyl, -3-hexenyl, -1-heptenyl, -2-heptenyl, -3-heptenyl, -1-octenyl, -2-octenyl, -3-octenyl, -1-nonenyl, -2-nonenyl, -3-nonenyl, -1-decenyl, -2-decenyl, -3-decenyl and the like.

The term "cycloalkenyl" refers to an unsaturated mono- or polycyclic hydrocarbon substituent with the indicated number of carbon atoms and containing at least one double carbon-carbon bond. Examples of a cycloalkenyl substituent include -cyclopentenyl, -cyclopentadienyl, -cyclohexenyl, -cyclohexadienyl, -cycloheptenyl, -cycloheptadienyl, -cycloheptatrienyl, -cyclooctenyl, -cyclooctadienyl, -cyclooctatrienyl, -cyclooctatetraenyl, -cyclononenyl, -cyclononadienyl, -cyclodecenyl, -cyclodecadienyl and the like.

The term "alkynyl" refers to an unsaturated, linear, or branched acyclic hydrocarbon substituent with the indicated number of carbon atoms and containing at least one triple carbon-carbon bond. Examples of an alkynyl substituent include -acetylenyl, -propynyl, -1-butynyl, -2-butynyl, -1-pentynyl, -2-pentynyl, -3-methyl-1-butynyl, -4-pentynyl, -1-hexynyl, -2-hexynyl, -5-hexynyl and the like.

The term "cycloalkynyl" refers to an unsaturated mono- or polycyclic hydrocarbon substituent with the indicated number of carbon atoms and containing at least one triple carbon-carbon bond.

Examples of a cycloalkynyl substituent include -cyclohexynyl, -cycloheptynyl, -cyclooctynyl, and the like.

The term "aryl" refers to an aromatic mono- or polycyclic hydrocarbon substituent with the indicated number of carbon atoms, possibly substituted with at least one alkyl, alkoxyl, aryloxyl, a halogen atom, hydroxyl group, nitro group, ester group or ketone group, cyanide group, an amide, carboxyl group, sulfonamide group, formyl group or ether group. Examples of an aryl substituent include -phenyl, -tolyl, -xylyl, -naphthyl, -2,4,6-trimethylphenyl, -2-fluorophenyl, -4-fluorophenyl, -2,4,6-trifluorophenyl, -2,6-difluoro-4-nitrophenyl and the like.

The term "aralkyl" refers to alkyl substituents as defined above, substituted with at least one aryl as defined above. Examples of an aralkyl substituent include -benzyl, -diphenylmethyl, -triphenylmethyl and the like.

The term "heteroaryl" refers to an aromatic mono- or polycyclic hydrocarbon substituent with the indicated number of carbon atoms, in which at least one carbon atom has been substituted by a heteroatom selected from among O, N and S. Examples of heteroaryl substituents include -furyl, -thienyl, -imidazolyl, -oxazolyl, -thiazolyl, -isoxazolyl, -triazolyl, -oxadiazolyl, -tiadiazolyl, -tetrazolyl, -pyridyl, -pyrimidyl, -triazynyl, -indolyl, -benzo[b]furyl, -benzo[b]tienyl, -indazolyl, -benzoimidazolyl, -azaindolyl, -quinolyl, -isoquinolyl, -carbazolyl and the like.

The term "aryloxyl" refers to an aryl substituent as defined above, connected via an oxygen atom.

The term "heteroaryloxyl" refers to a heteroacyl substituent as defined above connected via an oxygen atom.

The term "heterocycle" refers to a saturated or partially unsaturated, mono- or polycyclic hydrocarbon substituent, with the indicated number of carbon atoms, in which at least one carbon atom has been substituted by a heteroatom selected from among O, N and S.
Examples of a heterocyclic substituent include -furyl, -thiophenyl, -pyrolyl, -oxazolyl, -imidazolyl, - thiazolyl, -isoxazolyl, -pirazolyl, -isothiazolyl, -triazynyl, -pyrolidynonyl, -pyrolidynyl, -hydantoinyl, -oxiranyl, -oxethanyl, -tetrahydrofuranyl, -tetrahydrotiophenyl, -quinolinyl, -isoquinolinyl, -chromonyl, -cumarynyl, -indolyl, -indolizynyl, -benzo[b]furanyl, -benzo[b]tiophenyl, -indazolyl, -purynyl, -4H-quinolizynyl, -isoquinolyl, -quinolyl, -phthalazynyl, -naphthyrydynyl, -carbazolyl, -*β*-carbolinyl and the like.

The term "perfluoroaryl" denotes an aryl group as defined above in which all hydrogen atoms have been substituted by identical or different halogen atoms.

The term "neutral ligand" refers to a substituent devoid of charge, capable of coordinating with a metallic centre (ruthenium atom). Examples of such ligands may include: amines, phosphines and their oxides, phosphorines and alkyl and aryl phosphates, arsines and their oxides, ethers, aryl and alkyl sulphides, coordinated hydrocarbons, and alkyl and aryl halides.

The term "anionic ligand" refers to a substituent capable of coordinating with a metallic centre (ruthenium atom) possessing a charge, capable of partially or completely compensating the charge of the metallic centre. Examples of such ligands may be: such anions as fluoride, bromide, iodide, cyanide, cyanate and thiocyanate, carboxylic acid anions, alcohol anions, phenolic anions, thiol and thiophenol anions, hydrocarobon anions with a delocalised charge (i.e. cyclopentadiene), anions of (organo)sulphuric and (organo)phosphoric acids and esters thereof (such as i.e. anions of alkylsulphonic and arylsulphonic acids, anions of arylphsophoric and alkylphosphoric acids, anions of aryl and alkyl esters of sulphuric acids, anions of aryl and alkyl esters of phosphoric acids, anions of anions of aryl and alkyl esters of alkylphosphoric and arylophosphoric acids). Possibly, an anionic ligand may posses bound groups L₁ and L₂ such as a catechol anion, an acetylacetone anion or a salicyl aldehyde anion. The anionic ligands (X₁, X₂) and neutral ligands (L₁, L₂) may together form be may be connected to one another forming multidentate ligands, such as a: bidentate ligand (X₁, X₂), tridentate ligand (X₁, X₂, L₁), tetradentate ligand (X₁, X₂, L₁, L₂), bidentate ligand (X₁, L₁), tridentate ligand (X₁, L₁, L₂), or bidentate ligand (L₁, L₂). Examples of such ligands include a catechol anion, an acetylacetone anion as well as a salicylic aldehyde anion.
The examples below demonstrate the production and uses of the novel Complex **1**.

### Example I

The synthesis of catalysts defined by Formula Id (according to Scheme I)

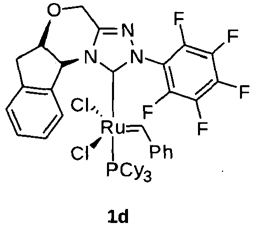

Using a protective atmosphere of argon, a Schlenk vessel was loaded with a carbene defined by Formula 3a (75.9 mg, 0.20 mmol) 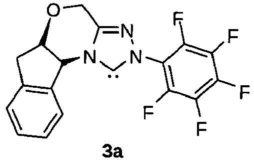 and dry deoxygenated toluene (4 mL) was added followed by solid carbene complex of ruthenium defined by Formula 2, in which X¹ and X² denote chlorine, L¹ and L² denotes tricyclohexylphosphine (PCy₃), R⁶ a hydrogen and R⁷ a phenyl (so-called Ist generation Grubbs catalyst, 164.6 mg, 0.20 mmol). The resulting solution was stirred at room temperature for 1 hour. From that moment onward, all subsequent operations were performed in the open air, without the need of a protective argon atmosphere. The reaction mixture was concentrated in an evaporator and loaded onto a chromatography column filled with a silica gel. The column was developed using an ethyl acetate-cyclohexane (10% v/v), and the brown fraction was collected. After evaporating off the solvent, we obtained Complex Id in the form of a brown, microcrystalline solid (112 mg, 61% yield).
MS (FD/FI) calculated for C₄₃H₄₉Cl₂F₅N₃OPRu: 921.2; found: 921.2;
¹H NMR (CD₂Cl₂, 600 MHz) = 19.80 (s), 7.67-7.61 (m), 7.38-7.30 (m), 7.30-7.15 (m), 5.10-4.60 (m), 3.40-2.70 (m), 2.40-2.30 (m), 1.85-1.35 (m), 1.16-1.00 (m) ppm.
¹³C NMR (CD₂Cl₂, 150 MHz) = 152.6, 129.4, 127.8, 126.8, 33.0 (m), 30.4, 28.3 (m), 26.8 (m) ppm.

### Example II

### The synthesis of catalysts defined by Formula 1d (according to Scheme I)

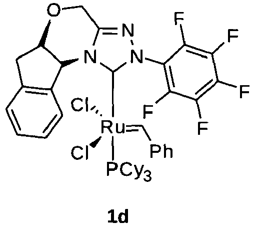

Using a protective atmosphere of argon, a Schlenk vessel was loaded with a complex of silver defined by Formula **4a** (114.4 mg, 0.12 mmol), in which R¹ denotes pentafluorophenyl 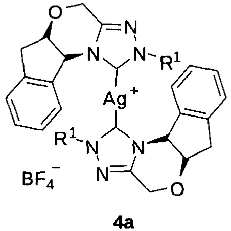 and dry deoxygenated toluene (4 mL) was added followed by solid carbene complexes of ruthenium defined by Formula 2, in which X¹ and X² denote chlorine, L¹ and L² denotes tricyclohexylphosphine (PCy₃), R⁶ a hydrogen and R⁷ a phenyl (so-called Ist generation Grubbs catalyst, 164.6 mg, 0.20 mmol). The resulting solution was stirred at room temperature for 1 hour. From that moment onward, all subsequent operations were performed in the open air, without the need of a protective argon atmosphere. The reaction mixture was concentrated in an evaporator and loaded onto a chromatography column filled with a silica gel. The column was developed using an ethyl acetate-cyclohexane (10% v/v), and the brown fraction was colletced. After evaporating off the solvent, we obtained Complex **1d** in the form of a brown, microcrystalline solid (108 mg, 59% yield).

### Example III

### The synthesis of catalysts defined by Formula Id (according to Scheme II)

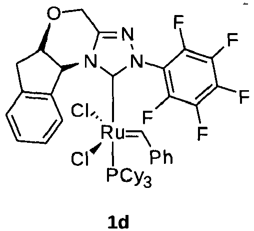

Using a protective atmosphere of argon, a Schlenk vessel was loaded with a carbene precursor defined by Formula **6a** (93.4 mg, 0.20 mmol) 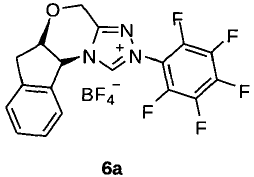 and dry deoxygenated toluene (4 mL) was added followed by potassium bis(trimethylsilyl)amide in toluene (0.5 M, 0.4 mL, 0.20 mmol). The resulting solution was stirred at room temperature for 15 minutes. Next, solid carbene complex of ruthenium defined by Formula **2,** in which X¹ and X² denote chlorine, L¹ and L² denotes tricyclohexylphosphine (PCy₃), R⁶ a hydrogen and R⁷ a phenyl (so-called Ist generation Grubbs catalyst, 164.6 mg, 0.20 mmol) was added. The resulting solution was stirred at room temperature for 1 hour. From that moment onward, all subsequent operations were performed in the open air, without the need of a protective argon atmosphere. The reaction mixture was concentrated in an evaporator and loaded onto a chromatography column filled with a silica gel. The column was developed using an ethyl acetate-cyclohexane (10% v/v), and the brown fraction was colleted. After evaporating off the solvent, we obtained Complex **1d** in the form of a brown, microcrystalline solid (87 mg, 47% yield).

### Example IV

### The synthesis of catalysts defined by Formula 1e (according to Scheme II)

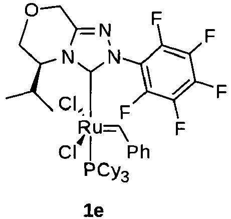

Using a protective atmosphere of argon, a Schlenk vessel was loaded with a carbene precursor defined by Formula **6b** (84.2 mg, 0.20 mmol) 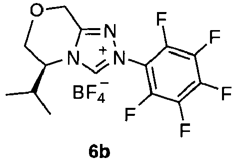 and dry deoxygenated toluene (4 mL) was added followed by potassium bis(trimethylsilyl)amide in toluene (0.5 M, 0.4 mL, 0.20 mmol). The resulting solution was stirred at room temperature for 15 minutes. Next, solid carbene complex of ruthenium defined by Formula 2, in which X¹ and X² denote chlorine, L¹ and L² denotes tricyclohexylphosphine (PCy₃), R⁶ a hydrogen and R⁷ phenyl (so-called Ist generation Grubbs catalyst, 164.6 mg, 0.20 mmol) was added. The resulting solution was stirred at room temperature for 1 hour. From that moment onward, all subsequent operations were performed in the open air, without the need of a protective argon atmosphere. The reaction mixture was concentrated in an evaporator and loaded onto a chromatography column filled with a silica gel. The column was developed using an ethyl acetate-cyclohexane (10% v/v), and the brown fraction was collected. After evaporating off the solvent, we obtained Complex **1e** in the form of a brown, microcrystalline solid (85 mg, 49% yield).
MS (FD/FI) calculated for C₃₉H₅₁Cl₂F₅N₃OPRu: 875.2; found: 875.2;
¹H NMR (toluen-d8, 400 MHz) = 20.39 (s), 7.30-7.14 (m), 4.46-4.28 (m), 4.01-3.89 (m), 3.80-3.72 (m), 3.59-3.52 (m), 3.22-3.12 (m), 2.90-2.75 (m), 2.50-2.30 (m), 2.05-1.80 (m), 1.80-1.10 (m), 0.87-0.77 (m) ppm.

### Example V

The synthesis of catalysts defined by Formula **1f** (according to Scheme I)

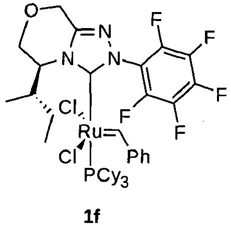

Using a protective atmosphere of argon, a Schlenk vessel was loaded with a complex of silver defined by Formula **4b** (106.7 mg, 0.12 mmol), in which R¹ denotes pentafluorophenyl 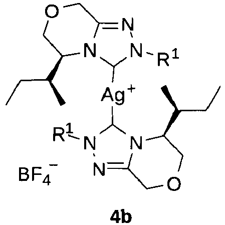 and dry deoxygenated tetrahydrofuran (4 mL) was added. Next, solid carbene complex of ruthenium defined by Formula 2, in which X¹ and X² denote chlorine, L¹ and L² denotes tricyclohexylphosphine (PCy₃), R⁶ a hydrogen and R⁷ phenyl (so-called Ist generation Grubbs catalyst, 164.6 mg, 0.20 mmol) was added. The resulting solution was stirred at room temperature for 1 hour. From that moment onward, all subsequent operations were performed in the open air, without the need of a protective argon atmosphere. The reaction mixture was concentrated in an evaporator and loaded onto a chromatography column filled with a silica gel. The column was developed using an ethyl acetate-cyclohexane (10% v/v), and the brown fraction was collected. After evaporating off the solvent, we obtained Complex **1f** in the form of a brown, microcrystalline solid (102 mg, 57% yield).
MS (FD/FI) calculated for C₄₀H₅₃Cl₂F₅N₃OPRu: 889.2; found: 889.2;
¹H NMR (benzen-d6, 600 MHz) = 20.34 (s), 8.72 (d, *J* = 7.6), 8.33-8.28 (m) 6.99-6.94 (m), 4.45 (d, *J* = 15.9), 4.00 (d, J = 15.9), 2.46-2.34 (m), 2.02-1.83 (m), 1.76-1.52 (m), 1.20-1.06 (m) ppm.
¹³C NMR (benzen-d6, 150 MHz) = 309.3, 189.0, 153.7, 153.0, 152.7, 150.6, 150.5, 131.8, 131.6, 130.7, 130.6, 129.7, 129.6, 128.7, 64.8, 64.7, 61.9, 61.8, 58.7, 57.4, 40.3, 36.2, 35.8, 33.6 (d, *J*_{CP} = 16), 32.8, 32.7, 32.6, 32.1, 32.0, 30.5, 30.3, 28.5, 28.4 (d, *J*_{CP} = 10), 27.6, 27.5, 27.4, 27.3, 27.2, 27.1, 26.9, 14.9, 14.5, 12.5, 12.2 ppm.

### Example VI

The use of complex 1 as (pre)catalysts for asymmetric ring opening metathesis with a cross metathesis (AROM/CM) of compound **S1**

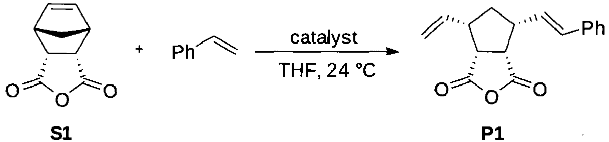

Using a protective atmosphere of argon, a Schlenk vessel was loaded with the substrate **S1** (32.8 mg, 0.2 mmol), styrene (41.7 mg, 0.4 mmol, 2 eq.) and dry deoxygenated tetrahydrofuran (1 mL). Next, solid carbene complex of ruthenium defined by Formula **1f** (0.004 mmol, 2 mol%) was added. The mixture was stirred at a temperature of 24 °C for 24 hours. After this time ethyl-vinyl ether (0.5 mL) was added and after 30 minutes mixture was evaporated. The product **P1** was isolated using column chromatography on a silica gel (ethyl acetate/cyclohexane = 1:4 v/v). The product was analyzed using high performance liquid chromatography with a chiral column (Chiralcel^{®} OJ, *n*-hexane/isopropanol = 1:1 v/v; 0.7 ml/min; 254 nm). Colourless solid (40 mg, 75%), ee = 67%.

### Example VII

The use of complex **1** as a (pre)catalyst for asymmetric ring opening metathesis with a cross metathesis (AROM/CM) of compound **S2**

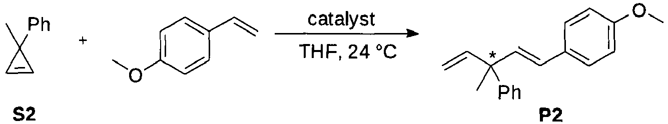

Using a protective atmosphere of argon, a Schlenk vessel was loaded with the substrate S2 (13.0 mg, 0.1 mmol), 4-vinyloanizol (26.8 mg, 0.2 mmol, 2 eq.) and dry deoxygenated tetrahydrofuran (0.5 mL). Next, solid carbene complex of ruthenium defined by Formula **1e** (0.005 mmol, 5 mol%) was added. The mixture was stirred at a temperature 24 °C for 24 hours. After this time ethyl-vinyl ether (0.5 mL) was added and after 30 minutes mixture was evaporated. The product **P2** was isolated using column chromatography on a silica gel (ethyl acetate/cyclohexane = 5:95 v/v). The product was analyzed using high performance liquid chromatography with chiral column (Chiralcel^{®} OD-H, *n*-hexane; 1.0 ml/min; 254 nm). Colourless oil (21 mg, 79%), ee = 48%.

## Claims

1. A ruthenium complex defined by Formula 1 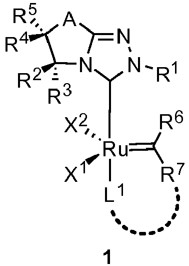 in which:
R¹ denotes a C₅-C₂₄ perfluoroaryl ;
R², R³, R⁴ and R⁵ independently of one another denote a hydrogen atom, a halogen atom, a C₁-C₂₅ alkyl, a C₃-C₇ cycloalkyl, a alkoxyl C₁-C₂₅, aryloxyl C₅-C₂₄, heteroaryloxyl C₅-C₂₀, a C₅-C₂₄ aryl, a C₅-C₂₀ heteroaryl, a C₇-C₂₄ aralkyl, a C₅-C₂₄ perfluoroaryl, a 3-12 membered heterocycle, wherein the groups R², R³, R⁴ and R⁵ may be mutually connected into a ring;
A denotes a -CH₂-, -O- or -OCH₂- group;
R⁶ and R⁷ independently of one another denote a hydrogen atom, a halogen atom, a C₁-C₂₅ alkyl, a C₁-C₂₅ perfluoroalkyl, a C₂-C₂₅ alkene, a C₃-C₇ cycloalkyl, a C₂-C₂₅ alkenyl, cycloalkenyl C₃-C₂₅, alkynyl C₂-C₂₅, cycloalkynyl C₃-C₂₅, a alkoxyl C₁-C₂₅, aryloxyl C₅-C₂₄, heteroaryloxyl C₅-C₂₀, a C₅-C₂₄ aryl, a C₅-C₂₀ heteroaryl, a C₇-C₂₄ aralkyl, a C₅-C₂₄ perfluoroaryl, or a 3-12 membered heterocycle wherein the alkyl groups may be mutually connected into a ring, wherein R⁶ and R⁷ preferably denote a hydrogen, aryl substituted with a nitro (-NO₂), cyanide (-CN), carboxyl (-COOH), ester (-COOR'), amide (-CONR'₂), sulfonyl (-SO₂R'), formyl (-CHO), sulfonamide (-SO₂NR'₂) or ketone (-COR') group, in which R' has the following meaning: a C₁-C₅ alkyl, a C₁-C₅ perfluoroalkyl, a C₅-C₂₄ aryl, a C₇-C₂₄ aralkyl, a C₅-C₂₄ perfluoroaryl;
L¹ denotes a neutral ligand selected from groups encompassing pyridine or substituted pyridine, P(R')₃, P(OR')₃, O(R')₂, N(R)₃, where each R' independently denotes a C₁-C₁₂ alkyl, a C₃-C₁₂ cycloalkyl, a C₅-C₂₀ aryl, a C₇-C₂₄ aralkyl, a C₅-C₂₄ perfluoroaryl, or a 5-12 membered heteroaryl;
X¹ and X² denote an anionic ligand independently selected from groups encompassing halide anions, the groups -CN, -SCN, -OR', -SR', -O(C=O)R', -O(SO₂)R', and -OSi(R')₃, where R' denotes a C₁-C₁₂ alkyl, a C₃-C₁₂ cycloalkyl, a C₂-C₁₂ alkenyl, or a C₅-C₂₀ aryl, which may possibly be substituted with at least one C₁-C₁₂ alkyl, a C₁-C₁₂ perfluoroalkyl, a C₁-C₁₂ alkoxyl. a C₅-C₂₄ aryloxyl, a C₅-C₂₀ heteroaryloxyl or a halogen atom,
wherein the term "perfluoroalkyl" denotes an alkyl group wherein all hydrogen atoms have been substituted by identical or different halide atoms, and
the term "perfluoroaryl" denotes an aryl group in which all hydrogen atoms have been substituted by identical or different halogen atoms.

2. A complex according to Claim 1, **characterised in that** it is a compound defined by Formula **1a** 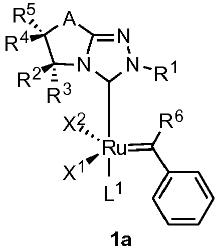 in which:
R¹, R², R³, R⁴, R⁵, A, L¹, X¹ and X² have the same meaning as in Formula 1;
R⁶ denotes a hydrogen.;

3. A complex according to Claim 1, **characterised in that it is** a compound defined by Formula **1b** 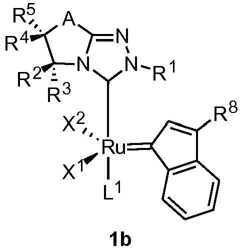 in which:
R¹, R², R³, R⁴, R⁵, A, L¹, X¹ and X² have the same meaning as in Formula 1;
R⁸ denotes a hydrogen atom, a C₅-C₂₀ aryl, a C₅-C₂₀ heteroaryl, a C₇-C₂₄ aralkyl, vinyl or allenyl.

4. A complex according to Claim 1, **characterised in that it is** a compound defined by Formula **1c** 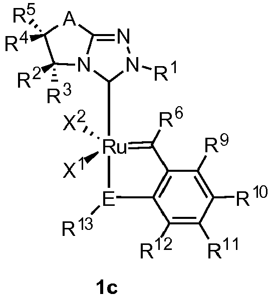 in which:
R¹, R², R³, R⁴, R⁵, A, L¹, X¹ and X² have the same meaning as in Formula **1**
R⁶ denotes a hydrogen
R⁹, R¹⁰, R¹¹, R¹² independently of one another denote a hydrogen atom, a halogen atom, a C₁-C₂₅ alkyl, a C₁-C₂₅ perfluoroalkyl, a C₂-C₂₅ alkene, a C₃-C₇ cycloalkyl, a C₂-C₂₅ alkenyl, cycloalkenyl C₃-C₂₅, alkynyl C₂-C₂₅, cycloalkynyl C₃-C₂₅, a C₅-C₂₄ aryl, a C₅-C₂₀ heteroaryl, a C₇-C₂₄ aralkyl, a C₅-C₂₄ perfluoroaryl, a 3-12 membered heterocycle wherein the alkyl groups may be mutually connected into a ring, an ether (-OR'), thioether (-SR'), nitro (-NO₂), cyanide (-CN), carboxyl (-COOH), ester (-COOR'), amide (-CONR'₂), imide (-CONR'COR'), amino (-NR'₂), amide (NR'COR'), sulfonamide (-NR'SO₂R'), sulfonyl (-SO₂R'), formyl (-CHO), sulfonamide (-SO₂NR'₂), or ketone (-COR') group, in which R' has the following meaning: a C₁-C₅ alkyl, a C₁-C₅ perfluoroalkyl, a C₅-C₂₄ aryl, a C₅-C₂₄ perfluoroaryl, a C₇-C₂₄ aralkyl, wherein the alkyl groups may be mutually connected into a ring, wherein R⁹, R¹⁰, R¹¹, R¹² preferably denotes a hydrogen;
R¹³ denotes a hydrogen atom, a C₁-C₂₅ alkyl, a C₁-C₂₅ perfluoroalkyl, a C₃-C₇ cycloalkyl, a C₅-C₂₄ aryl, a C₅-C₂₄ perfluoroaryl, a C₅-C₂₀ heteroaryl, a C₇-C₂₄ aralkyl, a 3-12 membered heterocycle wherein the alkyl groups may be mutually connected into a ring, a -COR' acyl, cyanide (-CN), carboxyl (-COOH), ester (-COOR'), amide (-CONR'₂), sulfonyl (-SO₂R'), formyl (-CHO), sulfonamide (-SO₂NR'₂), or ketone (-COR') group, in which R' has the following meaning: a C₁-C₅ alkyl, a C₁-C₅ perfluoroalkyl, a C₅-C₂₄ aryl, a C₅-C₂₄ perfluoroaryl, a C₇-C₂₄ aralkyl;
E denotes an oxygen atom.

5. A complex according to Claims from 1 to 4, **characterised in that**
R¹ denotes pentafluorophenyl;
R², R³, R⁴ and R⁵ independently of one another denote a hydrogen atom, a C₁-C₂₅ alkyl, a C₃-C₇ cycloalkyl, a C₅-C₂₄ aryl, a C₅-C₂₀ heteroaryl, a C₇-C₂₄ aralkyl, a C₅-C₂₄ perfluoroaryl, a 3-12 membered heterocycle, wherein the groups R², R³, R⁴ and R⁵ may be mutually connected into a ring;
A denotes a -CH₂-, -O- or -OCH₂- group;
L¹ denotes a neutral ligand selected from groups encompassing tricyclohexylphosphine, triphenylphosphine, pyridine, 3-bromopyridine;
X¹ and X² denote chlorine, bromine or iodine.

6. A method of producing a ruthenium complex defined in Claims from 1 to 5, **characterised in that** the carbene ruthenium complex defined by Formula 2 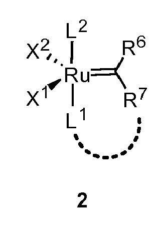 in which:
R⁶ and R⁷ independently of one another denote a hydrogen atom, a halogen atom, a C₁-C₂₅ alkyl, a C₁-C₂₅ perfluoroalkyl, a C₂-C₂₅ alkene, a C₃-C₇ cycloalkyl, a C₂-C₂₅ alkenyl, cycloalkenyl C₃-C₂₅, alkynyl C₂-C₂₅, cycloalkynyl C₃-C₂₅, a alkoxyl C₁-C₂₅, aryloxyl C₅-C₂₄, heteroaryloxyl C₅-C₂₀, a C₅-C₂₄ aryl, a C₅-C₂₀ heteroaryl, a C₇-C₂₄ aralkyl, a C₅-C₂₄ perfluoroaryl, a 3-12 membered heterocycle wherein the alkyl groups may be mutually connected into a ring, wherein R⁶ and R⁷ preferably denote a hydrogen, aryl substituted with a nitro (-NO₂), cyanide (-CN), carboxyl (-COOH), ester (-COOR'), amide (-CONR'₂), sulfonyl (-SO₂R'), formyl (-CHO), sulfonamide (-SO₂NR'₂) or ketone (-COR') group, in which R' has the following meaning: a C₁-C₅ alkyl, a C₁-C₅ perfluoroalkyl, a C₅-C₂₄ aryl, aC₇-C₂₄ aralkyl, a C₅-C₂₄ perfluoroaryl,
L¹ and L² denote a neutral ligand selected from groups encompassing pyridine or substituted pyridine, P(R')₃, P(OR')₃, O(R')₂, N(R')₃, where each R' independently denotes a C₁-C₁₂ alkyl, a C₃-C₁₂ cycloalkyl, a C₅-C₂₀ aryl, a C₇-C₂₄ aralkyl, a C₅-C₂₄ perfluoroaryl, or a 5-12 membered heteroaryl,
X¹ and X² denote an anionic ligand independently selected from groups encompassing halide anions, a -CN, -SCN, -OR', -SR', -O(C=O)R', -O(SO₂)R' or -OSi(R')₃ group, where R' denotes a C₁-C₁₂ alkyl, a C₃-C₁₂ cycloalkyl, a C₂-C₁₂ alkenyl, or a C₅-C₂₀ aryl, which may possibly be substituted with at least one C₁-C₁₂ alkyl, a C₁-C₁₂ perfluoroalkyl, a C₁-C₁₂ alkoxyl, a C₅-C₂₄ aryloxyl, a C₅-C₂₀ heteroaryloxyl or a halogen atom,
is subjected to a reaction with a chiral, non-racemic carbene defined by Formula 3 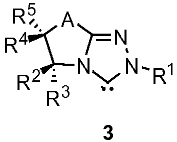
in which:
R¹ denotes a C₅-C₂₄ perfluoroaryl;
R², R³, R⁴ and R⁵ independently of one another denote a hydrogen atom, a halogen atom, a C₁-C₂₅ alkyl, a C₃-C₇ cycloalkyl, a alkoxyl C₁-C₂₅, aryloxyl C₅-C₂₄, heteroaryloxyl C₅-C₂₀, a C₅-C₂₄ aryl, a C₅-C₂₀ heteroaryl, a C₇-C₂₄ aralkyl, a C₅-C₂₄ perfluoroaryl, a 3-12 membered heterocycle, wherein the groups R², R³, R⁴ and R⁵ may be mutually connected into a ring;
A denotes a -CH₂-, -O- or -OCH₂- group.

7. A method of producing a ruthenium complex defined in Claims from 1 to 5, **characterised in that** the carbene ruthenium complex defined by Formula 2 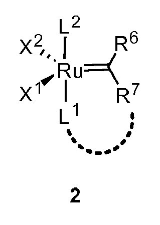 in which:
R⁶ and R⁷ independently of one another denote a hydrogen atom, a halogen atom, a C₁-C₂₅ alkyl, a C₁-C₂₅ perfluoroalkyl, a C₂-C₂₅ alkene, a C₃-C₇ cycloalkyl, a C₂-C₂₅ alkenyl, cycloalkenyl C₃-C₂₅, alkynyl C₂-C₂₅, cycloalkynyl C₃-C₂₅, a alkoxyl C₁-C₂₅, aryloxyl C₅-C₂₄, heteroaryloxyl C₅-C₂₀, a C₅-C₂₄ aryl, a C₅-C₂₀ heteroaryl, a C₇-C₂₄ aralkyl, a C₅-C₂₄ perfluoroaryl, a 3-12 membered heterocycle wherein the alkyl groups may be mutually connected into a ring, wherein R⁶ and R⁷ preferably denote a hydrogen, aryl substituted with a nitro (-NO₂), cyanide (-CN), carboxyl (-COOH), ester (-COOR'), amide (-CONR'₂), sulfonyl (-SO₂R'), formyl (-CHO), sulfonamide (-SO₂NR'₂) or ketone (-COR') group, in which R' has the following meaning: a C₁-C₅ alkyl, a C₁-C₅ perfluoroalkyl, a C₅-C₂₄ aryl, a C₇-C₂₄ aralkyl, a C₅-C₂₄ perfluoroaryl,
L¹ and L² denote a neutral ligand selected from groups encompassing pyridine or substituted pyridine, P(R')₃, P(OR')₃, O(R')₂, N(R')₃, where each R' independently denotes a C₁-C₁₂ alkyl, a C₃-C₁₂ cycloalkyl, a C₅-C₂₀ aryl, a C₇-C₂₄ aralkyl, a C₅-C₂₄ perfluoroaryl, or a 5-12 membered heteroaryl,
X¹ and X² denote an anionic ligand independently selected from groups encompassing halide anions, -CN, -SCN, -OR', -SR', -O(C=O)R', -O(SO₂)R', or -OSi(R')₃ groups where R' denotes a C₁-C₁₂ alkyl, a C₃-C₁₂ cycloalkyl, a C₂-C₁₂ alkenyl, or a C₅-C₂₀ aryl, which may possibly be substituted with at least one C₁-C₁₂ alkyl, a C₁-C₁₂ perfluoroalkyl, a C₁-C₁₂ alkoxyl, a C₅-C₂₄ aryloxyl, a C₅-C₂₀ heteroaryloxyl or a halogen atom,
is subjected to a reaction with a chiral, non-racemic complex of silver defined by Formula **4** 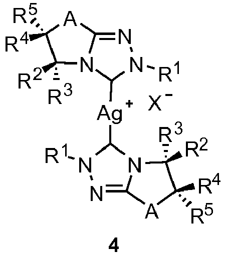 in which:
R¹ denotes C₅-C₂₄ perfluoroaryl;
R², R³, R⁴ and R⁵ independently of one another denote a hydrogen atom, a halogen atom, a C₁-C₂₅ alkyl, a C₃-C₇ cycloalkyl, a alkoxyl C₁-C₂₅, aryloxyl C₅-C₂₄, heteroaryloxyl C₅-C₂₀, a C₅-C₂₄ aryl, a C₅-C₂₀ heteroaryl, a C₇-C₂₄ aralkyl, a C₅-C₂₄ perfluoroaryl, a 3-12 membered heterocycle, wherein the groups R², R³, R⁴ and R⁵ may be mutually connected into a ring;
A denotes a -CH₂-, -O- or -OCH₂- group;
X denotes a halide anion or BF₄⁻, PF₆⁻ or ClO₄.

8. A method of producing a ruthenium complex defined in Claims 1-5, **characterised in that** the carbene ruthenium complex defined by Formula **2** 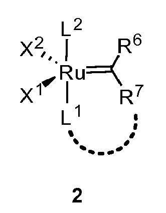 in which:
R⁶ and R⁷ independently of one another denote a hydrogen atom, a halogen atom, a C₁-C₂₅ alkyl, a C₁-C₂₅ perfluoroalkyl, a C₂-C₂₅ alkene, a C₃-C₇ cycloalkyl, a C₂-C₂₅ alkenyl, cycloalkenyl C₃-C₂₅, alkynyl C₂-C₂₅, cycloalkynyl C₃-C₂₅, a alkoxyl C₁-C₂₅, aryloxyl C₅-C₂₄, heteroaryloxyl C₅-C₂₀, a C₅-C₂₄ aryl, a C₅-C₂₀ heteroaryl, a C₇-C₂₄ aralkyl, a C₅-C₂₄ perfluoroaryl, a 3-12 membered heterocycle wherein the alkyl groups may be mutually connected into a ring, wherein R⁶ and R⁷ preferably denote a hydrogen, an aryl substituted with a nitro (-NO₂), cyanide (-CN), carboxyl (-COOH), ester (-COOR'), amide (-CONR'₂), sulfonyl (-SO₂R'), formyl (-CHO), sulfonamide (-SO₂NR'₂) or ketone (-COR') group, in which R' has the following meaning: a C₁-C₅ alkyl, a C₁-C₅ perfluoroalkyl, a C₅-C₂₄ aryl, a C₇-C₂₄ aralkyl, a C₅-C₂₄ perfluoroaryl,
L¹ and L² denote a neutral ligand selected from groups encompassing pyridine or substituted pyridine, P(R')₃, P(OR')₃, O(R')₂, N(R')₃, where each R' independently denotes a C₁-C₁₂ alkyl, a C₃-C₁₂ cycloalkyl, a C₅-C₂₀ aryl, a C₇-C₂₄ aralkyl, a C₅-C₂₄ perfluoroaryl, or a 5-12 membered heteroaryl,
X¹ and X² denote an anionic ligand independently selected from groups encompassing halide anions, a -CN, -SCN, -OR', -SR', -O(C=O)R', -O(SO₂)R', -OSi(R')₃ group, where R' denotes C₁-C₁₂ alkyl, a C₃-C₁₂ cycloalkyl, a C₂-C₁₂ alkenyl, or a C₅-C₂₀ aryl, which may possibly be substituted with at least one C₁-C₁₂ alkyl, a C₁-C₁₂ perfluoroalkyl, a C₁-C₁₂ alkoxyl, a C₅-C₂₄ aryloxyl, a C₅-C₂₀ heteroaryloxyl or a halogen atom,
is subjected to a reaction with a chiral, non racemic carbene precursor defined by Formula **5** 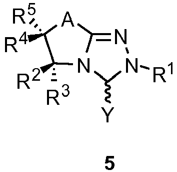 in which:
R¹ denotes C₅-C₂₄ perfluoroaryl ;
R², R³, R⁴ and R⁵ independently of one another denote a hydrogen atom, a halogen atom, a C₁-C₂₅ alkyl, a C₃-C₇ cycloalkyl, a alkoxyl C₁-C₂₅, aryloxyl C₅-C₂₄, heteroaryloxyl C₅-C₂₀, a C₅-C₂₄ aryl, a C₅-C₂₀ heteroaryl, a C₇-C₂₄ aralkyl, a C₅-C₂₄ perfluoroaryl, a 3-12 membered heterocycle, wherein the groups R², R³, R⁴ and R⁵ may be mutually connected into a ring;
A denotes a -CH₂-, -O- or -OCH₂- group;
Y denotes an alkoxyl, pentafluorophenyl or -CCl₃.

9. A method of producing a ruthenium complex defined in Claims from 1 to 5, **characterised in that** the carbene ruthenium complex defined by Formula **2** 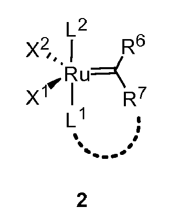 in which:
R⁶ and R⁷ independently of one another denote a hydrogen atom, a halogen atom, a C₁-C₂₅ alkyl, a C₁-C₂₅ perfluoroalkyl, a C₂-C₂₅ alkene, a C₃-C₇ cycloalkyl, a C₂-C₂₅ alkenyl, cycloalkenyl C₃-C₂₅, alkynyl C₂-C₂₅, cycloalkynyl C₃-C₂₅, a alkoxyl C₁-C₂₅, aryloxyl C₅-C₂₄, heteroaryloxyl C₅-C₂₀, a C₅-C₂₄ aryl, a C₅-C₂₀ heteroaryl, a C₇-C₂₄ aralkyl, a C₅-C₂₄ perfluoroaryl, a 3-12 membered heterocycle wherein the alkyl groups may be mutually connected into a ring, wherein R⁶ and R⁷ preferably denote a hydrogen, aryl substituted with a nitro (-NO₂), cyanide (-CN), carboxyl (-COOH), ester (-COOR'), amide (-CONR'₂), sulfonyl (-SO₂R'), formyl (-CHO), sulfonamide (-SO₂NR'₂) or ketone (-COR') group, in which R' has the following meaning: a C₁-C₅ alkyl, a C₁-C₅ perfluoroalkyl, a C₅-C₂₄ aryl, a C₇-C₂₄ aralkyl, a C₅-C₂₄ perfluoroaryl,
L¹ and L² denote a neutral ligand selected from groups encompassing pyridine or substituted pyridine, P(R')₃, P(OR')₃, O(R')₂, N(R')₃, where each R' independently denotes a C₁-C₁₂ alkyl, a C₃-C₁₂ cycloalkyl, a C₅-C₂₀ aryl, a C₇-C₂₄ aralkyl, a C₅-C₂₄ perfluoroaryl, or a 5-12 membered heteroaryl,
X¹ and X² denote an anionic ligand independently selected from groups encompassing halide anions, a -CN, -SCN, -OR', -SR', -O(C=O)R', -O(SO₂)R', -OSi(R')₃, where R' denotes C₁-C₁₂ alkyl, a C₃-C₁₂ cycloalkyl, a C₂-C₁₂ alkenyl, or a C₅-C₂₀ aryl, which may possibly be substituted with at least one C₁-C₁₂ alkyl, a C₁-C₁₂ perfluoroalkyl, a C₁-C₁₂ alkoxyl, a C₅-C₂₄ aryloxyl, a
C₅-C₂₀ heteroaryloxyl or a halogen atom,
is subjected to a reaction with a carbene formed as a result of the reaction of potassium tert-amylate or potassium tert-butanolate or potassium N,N-bis(trimethylsilyl)amide or sodium hydride with a chiral, non-racemic carbene precursor defined by Formula **6** 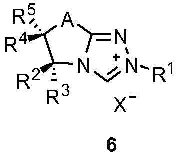 in which:
R¹ denotes C₅-C₂₄ perfluoroaryl;
R², R³, R⁴ and R⁵ independently of one another denote a hydrogen atom, a halogen atom, a C₁-C₂₅ alkyl, a C₃-C₇ cycloalkyl, a alkoxyl C₁-C₂₅, aryloxyl C₅-C₂₄, heteroaryloxyl C₅-C₂₀, a C₅-C₂₄ aryl, a C₅-C₂₀ heteroaryl, a C₇-C₂₄ aralkyl, a C₅-C₂₄ perfluoroaryl, a 3-12 membered heterocycle, wherein the groups R², R³, R⁴ and R⁵ may be mutually connected into a ring;
A denotes -CH₂-, -O- or -OCH₂- group;
X denotes a halide anion or BF₄⁻, PF₆⁻ or ClO₄⁻.

10. The method of producing a ruthenium complex according to any of Claims from 6 to 9, **characterised in that** as the carbene ruthenium complex use is made of a compound defined by Formula **2a** 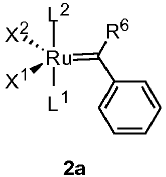 in which:
X¹, X², L¹ and L² have the same meaning as in Formula **2,**
R⁶ denotes a hydrogen.

11. The method of producing a ruthenium complex according to any of Claims from 6 to 9, **characterised in that** as the carbene ruthenium complex use is made of a compound defined by Formula **2b** 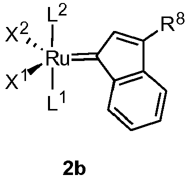 in which
X¹, X², L¹ and L² have the same meaning as in Formula **2,**
R⁸ denotes a hydrogen atom, a C₅-C₂₀ aryl, a C₅-C₂₀ heteroaryl, a C₇₋C₂₄ aralkyl, vinyl or allenyl.

12. The method of producing a ruthenium complex according to any of Claims from 6 to 9, **characterised in that** as the carbene ruthenium complex use is made of a compound defined by Formula **2c** 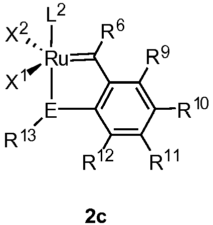 in which
X¹, X², L² have the same meaning as in Formula 2,
R⁶ denotes a hydrogen,
R⁹, R¹⁰, R¹¹, R¹² independently of one another denote a hydrogen atom, a halogen atom, a C₁-C₂₅ alkyl, a C₁-C₂₅ perfluoroalkyl, a C₂-C₂₅ alkene, a C₃-C₇ cycloalkyl, a C₂-C₂₅ alkenyl, cycloalkenyl C₃-C₂₅, alkynyl C₂-C₂₅, cycloalkynyl C₃-C₂₅, a C₅-C₂₄ aryl, a C₅-C₂₀ heteroaryl, a C₇-C₂₄ aralkyl, a C₅-C₂₄ perfluoroaryl, a 3-12 membered heterocycle wherein the alkyl groups may be mutually connected into a ring, an ether (-OR'), thioether (-SR'), nitro (-NO₂), cyanide (-CN), carboxyl (-COOH), ester (-COOR'), amide (-CONR'₂), imide (-CONR'COR'), amino (-NR'₂), amide (NR'COR'), sulfonamide (-NR'SO₂R'), sulfonyl (-SO₂R'), formyl (-CHO), sulfonamide (-SO₂NR'₂) or ketone (-COR') group, in which R' has the following meaning: a C₁-C₅ alkyl, a C₁-C₅ perfluoroalkyl, a C₅-C₂₄ aryl, a C₅-C₂₄ perfluoroaryl, aC₇₋C₂₄ aralkyl, wherein the alkyl groups may be mutually connected into a ring, wherein R⁹, R¹⁰, R¹¹ and R¹² preferably denotes a hydrogen;
R¹³ denotes a hydrogen atom, a C₁-C₂₅ alkyl, a C₁-C₂₅ perfluoroalkyl, a C₃-C₇ cycloalkyl, a C₅-C₂₄ aryl, a C₅-C₂₄ perfluoroaryl, a C₅-C₂₀ heteroaryl, a C₇-C₂₄ aralkyl, a 3-12 membered heterocycle wherein the alkyl groups may be mutually connected into a ring, a -COR' acyl group, cyanide (-CN), carboxyl (-COOH), ester (-COOR'), amide (-CONR'₂), sulfonyl (-SO₂R'), formyl (-CHO), sulfonamide (-SO₂NR'₂) or ketone (-COR') group, in which R' has the following meaning: a C₁-C₅ alkyl, a C₁-C₅ perfluoroalkyl, a C₅-C₂₄ aryl, a C₅-C₂₄ perfluoroaryl, a C₇-C₂₄ aralkyl, a C₅-C₂₄ perfluoroaryl;
E denotes an oxygen atom.

13. The method of producing a ruthenium complex according to any of Claims from 6 to 12, **characterised in that** the reaction is conducted in a protic or aprotic solvent, a chlorinated solvent or in an aromatic hydrocarbon solvent, or in mixtures thereof.

14. A use of a ruthenium complex defined by Formula 1 as defined in Claims from 1 to 5, as a (pre)catalyst in the metathesis and cycloisomerisation of olefins.

15. The use according to Claim 14, **characterized in that** use is made of the ruthenium complex as a (pre)catalyst in asymmetric ring opening metathesis with cross metathesis (AROM/CM), asymmetric cross metathesis (ACM), and asymmetric ring closure metathesis (ARCM).

## Patentansprüche

1. Rutheniumkomplex, wie definiert gemäß Formel 1 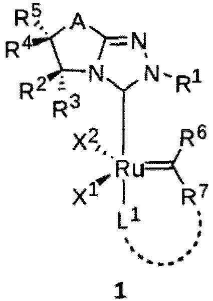 worin:
R¹ ein C₅-C₂₄ Perfluoraryl bezeichnet;
R², R³, R⁴ und R⁵ unabhängig voneinander ein Wasserstoffatom, ein Halogenatom, ein C₁-C₂₅ Alkyl, ein C₃-C₇ Cycloalkyl, ein Alkoxyl C₁-C₂₅, Aryloxyl C₅-C₂₄, Heteroaryloxyl C₅-C₂₀, ein C₅-C₂₄ Aryl, ein C₅-C₂₀ Heteroaryl, ein C₇-C₂₄ Aralkyl, ein C₅-C₂₄ Perfluoraryl, einen 3-12 Mitglieder zählenden Heterocyclus bezeichnen, wobei die Gruppen R², R³, R⁴ und R⁵ gemeinsam in einem Ring verbunden sein können;
A eine -CH₂-, -O- oder -OCH₂- Gruppe bezeichnet;
R⁶ und R⁷ unabhängig voneinander ein Wasserstoffatom, ein Halogenatom, ein C₁-C₂₅ Alkyl, ein C₁-C₂₅ Perfluoralkyl, ein C₂-C₂₅ Alken, ein C₃-C₇ Cycloalkyl, ein C₂-C₂₅ Alkenyl, Cycloalkenyl C₃-C₂₅, Alkinyl C₂-C₂₅, Cycloalkinyl C₃-C₂₅, ein Alkoxyl C₁-C₂₅, Aryloxyl C₅-C₂₄, Heteroaryloxyl C₅-C₂₀, ein C₅-C₂₄ Aryl, ein C₅-C₂₀ Heteroaryl, ein C₇-C₂₄ Aralkyl, ein C₅-C₂₄ Perfluoraryl, oder einen 3-12 Mitglieder zählenden Heterocyclus bezeichnen, wobei die Alkylgruppen gemeinsam in einem Ring verbunden sein können; worin R⁶ und R⁷ bevorzugt einen Wasserstoff, Aryl substituiert mit einer Nitro- (-NO₂), Cyanid- (-CN), Carboxyl- (-COOH), Ester- (-COOR'), Amid-(-CONR'₂), Sulfonyl- (-SO₂R'), Formyl- (-CHO), Sulfonamid- (-SO₂NR'₂ oder Keto- (-COR') Gruppe bezeichnet, in welcher R' die folgende Bedeutung hat: ein C₁-C₅ Alkyl, ein C₁-C₅ Perfluoralkyl, ein C₅-C₂₄ Aryl, ein C₇-C₂₄ Aralkyl, ein C₅-C₂₄ Perfluoraryl;
L¹ einen neutralen Liganden ausgewählt aus der Gruppe umfassend Pyridin oder substituiertes Pyridin, P(R')₃, P(OR')₃, O(R')₂, N(R')₃ bezeichnet, wobei jeder R' unabhängig ein C₁-C₁₂ Alkyl, ein C₃-C₁₂ Cycloalkyl, ein C₅-C₂₀ Aryl, ein C₇-C₂₄ Aralkyl, ein C₅-C₂₄ Perfluoraryl oder ein 5-12 Mitglieder zählendes Heteroaryl bezeichnet;
X¹ und X² einen anionischen Liganden unabhängig ausgewählt aus den Gruppen umfassend Halidanionen, die Gruppen -CN, -SCN, -OR', -SR', -O(C=O)R', -O(SO₂)R' und -OSi(R')₃ bezeichnen, wobei R' ein C₁-C₁₂ Alkyl, ein C₃-C₁₂ Cycloalkyl, ein C₂-C₁₂ Alkenyl oder ein C₅-C₂₀ Aryl bezeichnet, das, falls möglich, mit mindestens einem C₁-C₁₂ Alkyl, einem C₁-C₁₂ Perfluoralkyl, einem C₁-C₁₂ Alkoxyl, einem C₅-C₂₄ Aryloxyl, einem C₅-C₂₀ Heteroaryloxyl oder
einem Halogenatom substituiert sein kann,
wobei der Begriff "Perfluoralkyl" eine Alkylgruppe bezeichnet, worin alle Wasserstoffatome durch gleiche oder unterschiedliche Halidatome substituiert sind, und der Begriff "Perfluoraryl" eine Arylgruppe bezeichnet, worin alle Wasserstoffatome durch gleiche oder unterschiedliche Halogenatome substituiert sind.

2. Komplex nach Anspruch 1, **dadurch gekennzeichnet, dass** er eine Verbindung definiert gemäß Formel **1a** ist 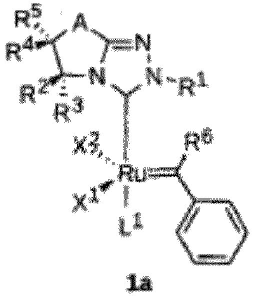 worin:
R¹, R², R³, R⁴, R⁵, A, L¹, X¹ und X² die gleiche Bedeutung wie in Formel 1 haben;
R⁶ ein Wasserstoff bezeichnet.

3. Komplex nach Anspruch 1, **dadurch gekennzeichnet, dass** er eine Verbindung definiert gemäß Formel **1b** ist 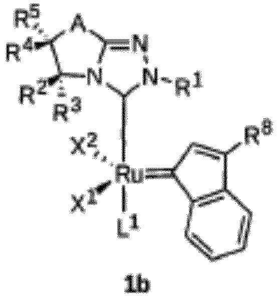 worin:
R¹, R², R³, R⁴, R⁵, A, L¹, X¹ und X² die gleiche Bedeutung wie in Formel 1 haben;
R⁸ ein Wasserstoff, ein C₅-C₂₀ Aryl, ein C₅-C₂₀ Heteroaryl, ein C₇-C₂₀ Aralkyl, Vinyl oder Allenyl bezeichnet.

4. Komplex nach Anspruch 1, **dadurch gekennzeichnet, dass** er eine Verbindung definiert gemäß Formel **1c** ist 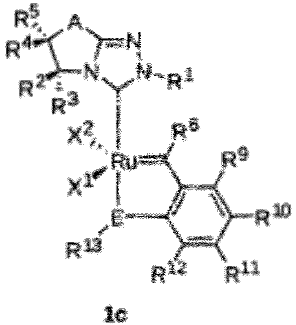 worin
R¹, R², R³, R⁴, R⁵, A, L¹, X¹ und X² die gleiche Bedeutung wie in Formel 1 haben;
R⁶ ein Wasserstoff bezeichnet,
R⁹, R¹⁰, R¹¹, R¹² unabhängig voneinander ein Wasserstoffatom, ein Halogenatom, ein C₁-C₂₅ Alkyl, ein C₁-C₂₅ Perfluoralkyl, ein C₂-C₂₅ Alken, ein C₃-C₇ Cycloalkyl, ein C₂-C₂₅ Alkenyl, Cycloalkenyl C₃-C₂₅, Alkinyl C₂-C₂₅, Cycloalkinyl C₃-C₂₅, ein C₅-C₂₄ Aryl, ein C₅-C₂₀ Heteroaryl, ein C₇₋C₂₄ Aralkyl, ein C₅-C₂₄ Perfluoraryl, oder einen 3-12 Mitglieder zählenden Heterocyclus bezeichnen, wobei die Alkylgruppen gemeinsam in einem Ring verbunden sein können, eine Ether- (-OR'), Thioether (-SR'), Nitro- (-NO₂), Cyanid- (-CN), Carboxyl- (-COOH), Ester- (-COOR'), Amid- (-CONR'₂), Imid- (-CONR'COR'), Amino- (-NR'₂), Amid- (-NR'COR'), Sulfonamid- (-NR'SO₂R'), Sulfonyl- (-SO₂R'), Formyl- (-CHO), ), Sulfonamid- (-SO₂NR'₂) oder Keto- (-COR') Gruppe bezeichnet, in welcher R' die folgende Bedeutung hat: ein C₁-C₅ Alkyl, ein C₁-C₅ Perfluoralkyl, ein C₅-C₂₄ Aryl, ein C₅-C₂₄ Perfluoraryl, ein C₇-C₂₄ Aralkyl; wobei die Alkylgruppen gemeinsam in einem Ring verbunden sein können; wobei R⁹, R¹⁰, R¹¹, R¹² bevorzugt einen Wasserstoff bezeichnen;
R¹³ ein Wasserstoffatom, ein C₁-C₂₅ Alkyl, ein C₁-C₂₅ Perfluoralkyl, ein C₃-C₇ Cycloalkyl, ein C₅-C₂₄ Aryl, ein C₅-C₂₄ Perfluoraryl, ein C₅-C₂₀ Heteroaryl, ein C₇-C₂₄ Aralkyl oder einen 3-12 Mitglieder zählenden Heterocyclus bezeichnet, wobei die Alkylgruppen gemeinsam in einem Ring verbunden sein können; eine -COR' Acyl-, Cyanid- (-CN), Carboxyl- (-COOH), Ester- (-COOR'), Amid- (-CONR'₂), Sulfonyl- (-SO₂R'), Formyl- (-CHO), Sulfonamid- (-SO₂NR'₂) oder Keto- (-COR') Gruppe bezeichnet, in welcher R' die folgende Bedeutung hat: ein C₁-C₅ Alkyl, ein C₁-C₅ Perfluoralkyl, ein C₅-C₂₄ Aryl, ein C₅-C₂₄ Perfluoraryl; ein C₇-C₂₄ Aralkyl, und
E ein Sauerstoffatom bezeichnet.

5. Komplex nach den Ansprüchen von 1 bis 4, **dadurch gekennzeichnet, dass**
R¹ Pentafluorphenyl bezeichnet,
R², R³, R⁴ und R⁵ unabhängig voneinander ein Wasserstoffatom, ein C₁-C₂₅ Alkyl, ein C₃-C₇ Cycloalkyl, ein C₅-C₂₄ Aryl, ein C₅-C₂₀ Heteroaryl, ein C₇-C₂₄ Aralkyl, ein C₅-C₂₄ Perfluoraryl, einen 3-12 Mitglieder zählenden Heterocyclus bezeichnen, wobei die Gruppen R², R³, R⁴ und R⁵ gemeinsam in einem Ring verbunden sein können;
A eine -CH₂-, -O- oder -OCH₂- Gruppe bezeichnet;
L¹ einen neutralen Liganden ausgewählt aus Gruppen umfassend Tricyclohexylphosphin, Triphenylphosphin, Pyridin, 3-Brompyridin bezeichnet;
X¹ und X² Chlor, Brom oder Jod bezeichnen.

6. Verfahren zur Herstellung eines Rutheniumkomplex wie definiert in Ansprüchen von 1 bis 5, **dadurch gekennzeichnet, dass** der Carben-Rutheniumkomplex definiert gemäß Formel **2** ist 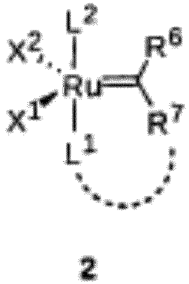 worin
R⁶ und R⁷ unabhängig voneinander ein Wasserstoffatom, ein Halogenatom, ein C₁-C₂₅ Alkyl, ein C₁-C₂₅ Perfluoralkyl, ein C₂-C₂₅ Alken, ein C₃-C₇ Cycloalkyl, ein C₂-C₂₅ Alkenyl, Cycloalkenyl C₃-C₂₅, Alkinyl C₂-C₂₅, Cycloalkinyl C₃-C₂₅, ein Alkoxyl C₁-C₂₅, Aryloxyl C₅-C₂₄, Heteroaryloxyl C₅-C₂₀, ein C₅-C₂₄ Aryl, ein C₅-C₂₀ Heteroaryl, ein C₇-C₂₄ Aralkyl, ein C₅-C₂₄ Perfluoraryl, oder einen 3-12 Mitglieder zählenden Heterocyclus bezeichnen, wobei die Alkylgruppen gemeinsam in einem Ring verbunden sein können; worin R⁶ und R⁷ bevorzugt einen Wasserstoff, Aryl substituiert mit einer Nitro- (-NO₂), Cyanid- (-CN), Carboxyl- (-COOH), Ester- (-COOR'), Amid-(-CONR'₂), Sulfonyl- (-SO₂R'), Formyl- (-CHO), Sulfonamid- (-SO₂NR'₂) oder Keto- (-COR') Gruppe bezeichnet, in welcher R' die folgende Bedeutung hat: ein C₁-C₅ Alkyl, ein C₁-C₅ Perfluoralkyl, ein C₅-C₂₄ Aryl, ein C₇-C₂₄ Aralkyl, ein C₅-C₂₄ Perfluoraryl;
L¹ und L² einen neutralen Liganden ausgewählt aus Gruppen umfassend Pyridin oder substituiertes Pyridin, P(R')₃, P(OR')₃, O(R')₂, N(R')₃ bezeichnen, wobei jeder R' unabhängig ein C₁-C₁₂ Alkyl, ein C₃-C₁₂ Cycloalkyl, ein C₅-C₂₀ Aryl, ein C₇C₂₄Aralkyl, ein C₅-C₂₄ Perfluoraryl oder ein 5-12 Mitglieder zählendes Heteroaryl bezeichnet;
X¹ und X² einen anionischen Liganden unabhängig ausgewählt aus Gruppen umfassend Halidanionen, einer Gruppe -CN, -SCN, -OR', -SR', -O(C=O)R', -O(SO₂)R' oder -OSi(R')₃ bezeichnen, wobei R' ein C₁-C₁₂ Alkyl, ein C₃-C₁₂ Cycloalkyl, ein C₂-C₁₂ Alkenyl oder ein C₅-C₂₀ Aryl bezeichnet, das, falls möglich, mit mindestens einem C₁-C₁₂ Alkyl, einem C₁-C₁₂ Perfluoralkyl, einem C₁-C₁₂ Alkoxyl, einem C₅-C₂₄ Aryloxyl, einem C₅-C₂₀ Heteroaryloxyl oder einem Halogenatom substituiert sein kann,
einer Reaktion mit einem chiralen, nicht-racemischem Carben wie definiert gemäß Formel **3** unterzogen wird 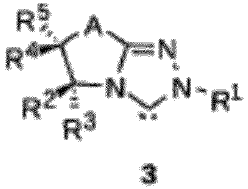 worin:
R¹ ein C₅-C₂₄ Perfluoraryl bezeichnet;
R², R³, R⁴ und R⁵ unabhängig voneinander ein Wasserstoffatom, ein Halogenatom, ein C₁-C₂₅ Alkyl, ein C₃-C₇ Cycloalkyl, ein Alkoxyl C₁-C₂₅, Aryloxyl C₅-C₂₄, Heteroaryloxyl C₅-C₂₀, ein C₅-C₂₄ Aryl, ein C₅-C₂₀ Heteroaryl, ein C₇-C₂₄ Aralkyl, ein C₅-C₂₄ Perfluoraryl, einen 3-12 Mitglieder zählenden Heterocyclus bezeichnen, wobei die Gruppen R², R³, R⁴ und R⁵ gemeinsam in einem Ring verbunden sein können;
A eine -CH₂-, -O- oder -OCH₂- Gruppe bezeichnet.

7. Verfahren zur Herstellung eines Rutheniumkomplex wie definiert in Ansprüchen von 1 bis 5, **dadurch gekennzeichnet, dass** der Carben-Rutheniumkomplex definiert gemäß Formel **2** ist 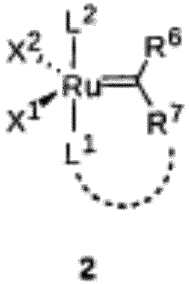 worin
R⁶ und R⁷ unabhängig voneinander ein Wasserstoffatom, ein Halogenatom, ein C₁-C₂₅ Alkyl, ein C₁-C₂₅ Perfluoralkyl, ein C₂-C₂₅ Alken, ein C₃-C₇ Cycloalkyl, ein C₂-C₂₅ Alkenyl, Cycloalkenyl C₃-C₂₅, Alkinyl C₂-C₂₅, Cycloalkinyl C₃-C₂₅, ein Alkoxyl C₁-C₂₅, Aryloxyl C₅-C₂₄, Heteroaryloxyl C₅-C₂₀, ein C₅-C₂₄ Aryl, ein C₅-C₂₀ Heteroaryl, ein C₇-C₂₄ Aralkyl, ein C₅-C₂₄ Perfluoraryl, oder einen 3-12 Mitglieder zählenden Heterocyclus bezeichnen, wobei die Alkylgruppen gemeinsam in einem Ring verbunden sein können; worin R⁶ und R⁷ bevorzugt einen Wasserstoff, Aryl substituiert mit einer Nitro- (-NO₂), Cyanid- (-CN), Carboxyl- (-COOH), Ester- (-COOR'), Amid-(-CONR'₂), Sulfonyl- (-SO₂R'), Formyl- (-CHO), Sulfonamid- (-SO₂NR'₂) oder Keto- (-COR') Gruppe bezeichnet, in welcher R' die folgende Bedeutung hat: ein C₁-C₅ Alkyl, ein C₁-C₅ Perfluoralkyl, ein C₅-C₂₄ Aryl, ein C₇-C₂₄ Aralkyl, ein C₅-C₂₄ Perfluoraryl;
L¹ und L² einen neutralen Liganden ausgewählt aus Gruppen umfassend Pyridin oder substituiertes Pyridin, P(R')₃, P(OR')₃, O(R')₂, N(R')₃ bezeichnen, wobei jeder R' unabhängig ein C₁-C₁₂ Alkyl, ein C₃-C₁₂ Cycloalkyl, ein C₅-C₂₀ Aryl, ein C₇-C₂₄ Aralkyl, ein C₅-C₂₄ Perfluoraryl oder ein 5-12 Mitglieder zählendes Heteroaryl bezeichnet;
X¹ und X² einen anionischen Liganden unabhängig ausgewählt aus Gruppen umfassend Halidanionen, einer Gruppe -CN, -SCN, -OR', -SR', -O(C=O)R', -O(SO₂)R' oder -OSi(R')₃ bezeichnen, wobei R' ein C₁-C₁₂ Alkyl, ein C₃-C₁₂ Cycloalkyl, ein C₂-C₁₂ Alkenyl oder ein C₅-C₂₀ Aryl bezeichnet, das, falls möglich, mit mindestens einem C₁-C₁₂ Alkyl, einem C₁-C₁₂ Perfluoralkyl, einem C₁-C₁₂ Alkoxyl, einem C₅-C₂₄ Aryloxyl, einem C₅-C₂₀ Heteroaryloxyl oder einem Halogenatom substituiert sein kann,
einer Reaktion mit einem chiralen, nicht-racemischem Komplex von Silber wie definiert gemäß Formel **4** unterzogen wird 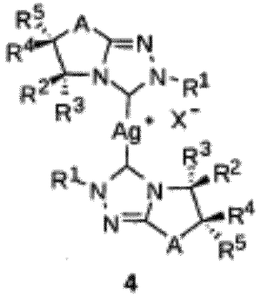 worin:
R¹ ein C₅-C₂₄ Perfluoraryl bezeichnet;
R², R³, R⁴ und R⁵ unabhängig voneinander ein Wasserstoffatom, ein Halogenatom, ein C₁-C₂₅ Alkyl, ein C₃-C₇ Cycloalkyl, ein Alkoxyl C₁-C₂₅, Aryloxyl C₅-C₂₄, Heteroaryloxyl C₅-C₂₀, ein C₅-C₂₄ Aryl, ein C₅-C₂₀ Heteroaryl, ein C₇-C₂₄ Aralkyl, ein C₅-C₂₄ Perfluoraryl, einen 3-12 Mitglieder zählenden Heterocyclus bezeichnen, wobei die Gruppen R², R³, R⁴ und R⁵ gemeinsam in einem Ring verbunden sein können;
A eine -CH₂-, -O- oder -OCH₂- Gruppe bezeichnet,
X ein Halidanion oder BF₄⁻, PF₆⁻ oder ClO₄⁻ bezeichnet.

8. Verfahren zur Herstellung eines Rutheniumkomplex wie definiert in Ansprüchen von 1 bis 5, **dadurch gekennzeichnet, dass** der Carben-Rutheniumkomplex definiert gemäß Formel 2 ist 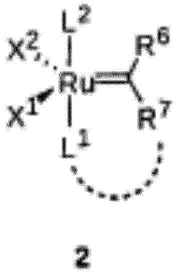 worin:
R⁶ und R⁷ unabhängig voneinander ein Wasserstoffatom, ein Halogenatom, ein C₁-C₂₅ Alkyl, ein C₁-C₂₅ Perfluoralkyl, ein C₂-C₂₅ Alken, ein C₃-C₇ Cycloalkyl, ein C₂-C₂₅ Alkenyl, Cycloalkenyl C₃-C₂₅, Alkinyl C₂-C₂₅, Cycloalkinyl C₃-C₂₅, ein Alkoxyl C₁-C₂₅, Aryloxyl C₅-C₂₄, Heteroaryloxyl C₅-C₂₀, ein C₅-C₂₄ Aryl, ein C₅-C₂₀ Heteroaryl, ein C₇-C₂₄ Aralkyl, ein C₅-C₂₄ Perfluoraryl, oder einen 3-12 Mitglieder zählenden Heterocyclus bezeichnen, wobei die Alkylgruppen gemeinsam in einem Ring verbunden sein können; worin R⁶ und R⁷ bevorzugt einen Wasserstoff, Aryl substituiert mit einer Nitro- (-NO₂), Cyanid- (-CN), Carboxyl- (-COOH), Ester- (-COOR'), Amid-(-CONR'₂), Sulfonyl- (-SO₂R'), Formyl- (-CHO), Sulfonamid- (-SO₂NR'₂) oder Keto- (-COR') Gruppe bezeichnet, in welcher R' die folgende Bedeutung hat: ein C₁-C₅ Alkyl, ein C₁-C₅ Perfluoralkyl, ein C₅-C₂₄ Aryl, ein C₇-C₂₄ Aralkyl, ein C₅-C₂₄ Perfluoraryl;
L¹ und L² einen neutralen Liganden ausgewählt aus Gruppen umfassend Pyridin oder substituiertes Pyridin, P(R')₃, P(OR')₃, O(R')₂, N(R')₃ bezeichnen, wobei jeder R' unabhängig ein C₁-C₁₂ Alkyl, ein C₃-C₁₂ Cycloalkyl, ein C₅-C₂₀ Aryl, ein C₇-C₂₄ Aralkyl, ein C₅-C₂₄ Perfluoraryl oder ein 5-12 Mitglieder zählendes Heteroaryl bezeichnet;
X¹ und X² einen anionischen Liganden unabhängig ausgewählt aus Gruppen umfassend Halidanionen, einer Gruppe -CN, -SCN, -OR', -SR', -O(C=O)R', -O(SO₂)R' oder -OSi(R')₃ bezeichnen, wobei R' ein C₁-C₁₂ Alkyl, ein C₃-C₁₂ Cycloalkyl, ein C₂-C₁₂ Alkenyl oder ein C₅-C₂₀ Aryl bezeichnet, das, falls möglich, mit mindestens einem C₁-C₁₂ Alkyl, einem C₁-C₁₂ Perfluoralkyl, einem C₁-C₁₂ Alkoxyl, einem C₅-C₂₄ Aryloxyl, einem C₅-C₂₀ Heteroaryloxyl oder einem Halogenatom substituiert sein kann,
einer Reaktion mit einem chiralen, nicht-racemischem Carbenvorläufer wie definiert gemäß Formel **5** unterzogen wird 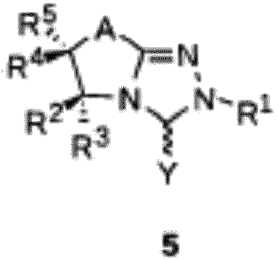 worin:
R¹ ein C₅-C₂₄ Perfluoraryl bezeichnet;
R², R³, R⁴ und R⁵ unabhängig voneinander ein Wasserstoffatom, ein Halogenatom, ein C₁-C₂₅ Alkyl, ein C₃-C₇ Cycloalkyl, ein Alkoxyl C₁-C₂₅, Aryloxyl C₅-C₂₄, Heteroaryloxyl C₅-C₂₀, ein C₅-C₂₄ Aryl, ein C₅-C₂₀ Heteroaryl, ein C₇-C₂₄ Aralkyl, ein C₅-C₂₄ Perfluoraryl, einen 3-12 Mitglieder zählenden Heterocyclus bezeichnen, wobei die Gruppen R², R³, R⁴ und R⁵ gemeinsam in einem Ring verbunden sein können;
A eine -CH₂-, -O- oder -OCH₂- Gruppe bezeichnet;
Y ein Alkoxyl, Pentafluorphenyl oder -CCl₃ bezeichnet.

9. Verfahren zur Herstellung eines Rutheniumkomplex wie definiert in Ansprüchen von 1 bis 5, **dadurch gekennzeichnet, dass** der Carben-Rutheniumkomplex definiert gemäß Formel 2 ist 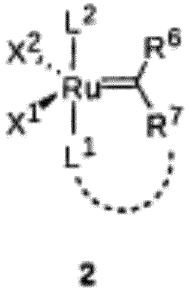 worin:
R⁶ und R⁷ unabhängig voneinander ein Wasserstoffatom, ein Halogenatom, ein C₁-C₂₅ Alkyl, ein C₁-C₂₅ Perfluoralkyl, ein C₂-C₂₅ Alken, ein C₃-C₇ Cycloalkyl, ein C₂-C₂₅ Alkenyl, Cycloalkenyl C₃-C₂₅, Alkinyl C₂-C₂₅, Cycloalkinyl C₃-C₂₅, ein Alkoxyl C₁-C₂₅, Aryloxyl C₅-C₂₄, Heteroaryloxyl C₅-C₂₀, ein C₅-C₂₄ Aryl, ein C₅-C₂₀ Heteroaryl, ein C₇-C₂₄ Aralkyl, ein C₅-C₂₄ Perfluoraryl, oder einen 3-12 Mitglieder zählenden Heterocyclus bezeichnen, wobei die Alkylgruppen gemeinsam in einem Ring verbunden sein können; worin R⁶ und R⁷ bevorzugt einen Wasserstoff, Aryl substituiert mit einer Nitro- (-NO₂), Cyanid- (-CN), Carboxyl- (-COOH), Ester- (-COOR'), Amid-(-CONR'₂), Sulfonyl- (-SO₂R'), Formyl- (-CHO), Sulfonamid- (-SO₂NR'₂) oder Keto- (-COR') Gruppe bezeichnet, in welcher R' die folgende Bedeutung hat: ein C₁-C₅ Alkyl, ein C₁-C₅ Perfluoralkyl, ein C₅-C₂₄ Aryl, ein C₇-C₂₄ Aralkyl, ein C₅-C₂₄ Perfluoraryl;
L¹ und L² einen neutralen Liganden ausgewählt aus Gruppen umfassend Pyridin oder substituiertes Pyridin, P(R')₃, P(OR')₃, O(R')₂, N(R')₃ bezeichnen, wobei jeder R' unabhängig ein C₁-C₁₂ Alkyl, ein C₃-C₁₂ Cycloalkyl, ein C₅-C₂₀ Aryl, ein C₇-C₂₄ Aralkyl, ein C₅-C₂₄ Perfluoraryl oder ein 5-12 Mitglieder zählendes Heteroaryl bezeichnet;
X¹ und X² einen anionischen Liganden unabhängig ausgewählt aus Gruppen umfassend Halidanionen, einer Gruppe -CN, -SCN, -OR', -SR', -O(C=O)R', -O(SO₂)R' oder -OSi(R')₃ bezeichnen, wobei R' ein C₁-C₁₂ Alkyl, ein C₃-C₁₂ Cycloalkyl, ein C₂-C₁₂ Alkenyl oder ein C₅-C₂₀ Aryl bezeichnet, das, falls möglich, mit mindestens einem C₁-C₁₂ Alkyl, einem C₁-C₁₂ Perfluoralkyl, einem C₁-C₁₂ Alkoxyl, einem C₅-C₂₄ Aryloxyl, einem C₅-C₂₀ Heteroaryloxyl oder einem Halogenatom substituiert sein kann,
einer Reaktion mit einem Carben unterzogen wird, das als Ergebnis der Reaktion von tert-Amylat oder Kalium tert-Butanolat oder Kalium N,N-bis(Trimethylsilyl)amid oder Natriumhydrid mit einem chiralen nicht-racemischem Carbenvorläufer wie definiert gemäß Formel **6** gebildet wurde 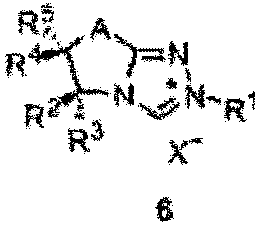 worin
R¹ ein C₅-C₂₄ Perfluoraryl bezeichnet;
R², R³, R⁴ und R⁵ unabhängig voneinander ein Wasserstoffatom, ein Halogenatom, ein C₁-C₂₅ Alkyl, ein C₃-C₇ Cycloalkyl, ein Alkoxyl C₁-C₂₅, Aryloxyl C₅-C₂₄, Heteroaryloxyl C₅-C₂₀, ein C₅-C₂₄ Aryl, ein C₅-C₂₀ Heteroaryl, ein C₇-C₂₄ Aralkyl, ein C₅-C₂₄ Perfluoraryl, einen 3-12 Mitglieder zählenden Heterocyclus bezeichnen, wobei die Gruppen R², R³, R⁴ und R⁵ gemeinsam in einem Ring verbunden sein können;
A eine -CH₂-, -O- oder -OCH₂- Gruppe bezeichnet,
X ein Halidanion oder BF₄⁻, PF₆⁻ oder ClO₄⁻ bezeichnet.

10. Verfahren zur Herstellung eines Rutheniumkomplex nach einem der Ansprüche von 6 bis 9, **dadurch gekennzeichnet, dass** als der Carben-Rutheniumkomplex eine Verbindung wie definiert gemäß Formel 2a verwendet wird 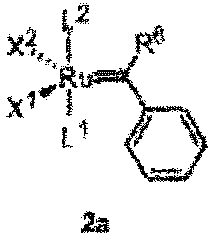 worin
X¹, X², L¹ und L² die gleiche Bedeutung wie in Formel 2 haben;
R⁶ ein Wasserstoff bezeichnet.

11. Verfahren zur Herstellung eines Rutheniumkomplex nach einem der Ansprüche von 6 bis 9, **dadurch gekennzeichnet, dass** als der Carben-Rutheniumkomplex eine Verbindung wie definiert gemäß Formel **2b** verwendet wird 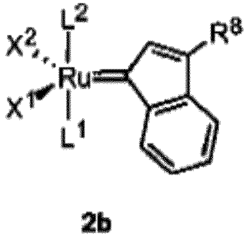 worin
X¹, X², L¹ und L² die gleiche Bedeutung wie in Formel 2 haben;
R⁶ ein Wasserstoff, ein C₅-C₂₀ Aryl, ein C₅-C₂₀ Heteroaryl, ein C₇-C₂₄ Aralkyl, Vinyl oder Allenyl bezeichnet.

12. Verfahren zur Herstellung eines Rutheniumkomplex nach einem der Ansprüche von 6 bis 9, **dadurch gekennzeichnet, dass** als der Carben-Rutheniumkomplex eine Verbindung wie definiert gemäß Formel 2c verwendet wird 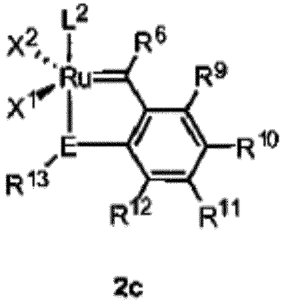 worin
X¹, X², L² die gleiche Bedeutung wie in Formel 2 haben;
R⁶ ein Wasserstoff bezeichnet,
R⁹, R¹⁰, R¹¹, R¹² unabhängig voneinander ein Wasserstoffatom, ein Halogenatom, ein C₁-C₂₅ Alkyl, ein C₁-C₂₅ Perfluoralkyl, ein C₂-C₂₅ Alken, ein C₃-C₇ Cycloalkyl, ein C₂-C₂₅ Alkenyl, Cycloalkenyl C₃-C₂₅, Alkinyl C₂-C₂₅, Cycloalkinyl C₃-C₂₅, ein C₅-C₂₄ Aryl, ein C₅-C₂₀ Heteroaryl, ein C₇-C₂₄ Aralkyl, ein C₅-C₂₄ Perfluoraryl, oder einen 3-12 Mitglieder zählenden Heterocyclus bezeichnen, wobei die Alkylgruppen gemeinsam in einem Ring verbunden sein können, eine Ether- (-OR'), Thioether (-SR'), Nitro- (-NO₂), Cyanid- (-CN), Carboxyl- (-COOH), Ester- (-COOR'), Amid- (-CONR'₂), Imid- (-CONR'COR'), Amino- (-NR'₂), Amid- (-NR'COR'), Sulfonamid- (-NR'SO₂R'), Sulfonyl- (-SO₂R'), Formyl- (-CHO), Sulfonamid- (-SO₂NR'₂) oder Keto- (-COR') Gruppe bezeichnet, in welcher R' die folgende Bedeutung hat: ein C₁-C₅ Alkyl,
ein C₁-C₅ Perfluoralkyl, ein C₅-C₂₄ Aryl, ein C₅-C₂₄ Perfluoraryl, ein C₇-C₂₄ Aralkyl; wobei die Alkylgruppen gemeinsam in einem Ring verbunden sein können, worin R⁹, R¹⁰, R¹¹, R¹² bevorzugt Wasserstoff bezeichnet;
R¹³ ein Wasserstoffatom, ein C₁-C₂₅ Alkyl, ein C₁-C₂₅ Perfluoralkyl, ein C₃-C₇ Cycloalkyl, ein C₂-C₂₄ Aryl, ein C₅-C₂₄ Perfluoraryl, ein C₅-C₂₀ Heteroaryl, ein C₇-C₂₄ Aralkyl, oder einen 3-12 Mitglieder zählenden Heterocyclus bezeichnet, wobei die Alkylgruppen gemeinsam in einem Ring verbunden sein können, eine -COR' Acylgruppe, eine Cyanid- (-CN), Carboxyl- (-COOH), Ester- (-COOR'), Amid- (-CONR'₂), Sulfonyl- (-SO₂R'), Formyl- (-CHO), ), Sulfonamid- (-SO₂NR'₂) oder Keto- (-COR') Gruppe bezeichnet, in welcher R' die folgende Bedeutung hat: ein C₁-C₅ Alkyl, ein C₁-C₅ Perfluoralkyl, ein C₅-C₂₄ Aryl, ein C₅-C₂₄ Perfluoraryl, ein C₇-C₂₄ Aralkyl, ein C₅-C₂₄ Perfluoraryl;
E ein Sauerstoffatom bezeichnet.

13. Verfahren zur Herstellung eines Rutheniumkomplex nach einem der Ansprüche von 6 bis 12, **dadurch gekennzeichnet, dass** die Reaktion in einem protischen oder aprotischen Lösemittel, einem chlorinierten Lösemittel oder in einem aromatischen Kohlenwasserstoff-Lösemittel oder in Gemischen davon durchgeführt wird.

14. Verwendung eines Rutheniumkomplexes definiert gemäß Formel **1** nach einem der Ansprüche von 1 bis 5 als ein (Vor)Katalysator in der Methathese und Cycloisomerisierung von Olefinen.

15. Verwendung nach Anspruch 14, **dadurch gekennzeichnet, dass** der Rutheniumkomplex als ein (Vor)Katalysator in einer asymmetrischen Ringöffnungsmethathese mit Kreuzmethathese (AROM/CM), asymmetrischen Kreuzmethathese (ACM) und asymmetrischen Ringschlussmethathese (ARCM) verwendet wird.

## Revendications

1. Complexe de ruthénium défini par la formule 1 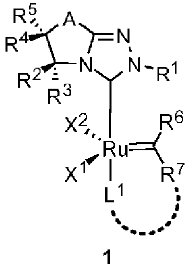 dans lequel :
R¹ désigne un perfluoroaryle en C₅-C₂₄ ;
R², R³, R⁴ et R⁵ indépendamment les uns des autres désignent un atome d'hydrogène, un atome d'halogène, un alkyle en C₁-C₂₅, un cycloalkyle en C₃-C₇, un alcoxyle en C₁-C₂₅, un aryloxyle en C₅-C₂₄, un hétéroaryloxyle en C₅-C₂₀, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons, où les groupes R², R³, R⁴ et R⁵ peuvent être mutuellement reliés dans un cycle ;
A désigne un groupe -CH₂-, -O- ou -OCH₂- ;
R⁶ et R⁷ indépendamment l'un de l'autre désignent un atome d'hydrogène, un atome d'halogène, un alkyle en C₁-C₂₅, un perfluoroalkyle en C₁-C₂₅, un alcène en C₂-C₂₅, un cycloalkyle en C₃-C₇, un alcényle en C₂-C₂₅, un cycloalcényle en C₃-C₂₅, un alcynyle en C₂-C₂₅, un cycloalcynyle en C₃-C₂₅, un alcoxyle en C₁-C₂₅, un aryloxyle en C₅-C₂₄, un hétéroaryloxyle en C₅-C₂₀, un aryle en C₅-C₂₄, un hétéroaryle en C₅-C₂₀, un aralkyle en C₇-C₂₄, un perfluoroaryle en C₅-C₂₄, ou un hétérocycle de 3 à 12 chaînons où les groupes alkyle peuvent être mutuellement reliés dans un cycle, où R⁶ et R⁷ désignent de préférence hydrogène, aryle substitué par un groupe nitro (-NO₂), cyanure (-CN), carboxyle (-COOH), ester (-COOR'), amide (-CONR'₂), sulfonyle (-SO₂R'), formyle (-CHO), sulfonamide (-SO₂NR'₂) ou cétone (-COR'), où R' a la définition suivante : un alkyle en C₁-C₅, un perfluoroalkyle en C₁-C₅, un aryle en C₅-C₂₄, un aralkyle en C₇-C₂₄, un perfluoroaryle en C₅-C₂₄ ;
L¹ désigne un ligand neutre choisi parmi les groupes comprenant pyridine ou pyridine substituée, P(R')₃, P(OR')₃, O(R')₂, N(R')₃, où chaque R' indépendamment désigne un alkyle en C₁-C₁₂, un cycloalkyle en C₃-C₁₂, un aryle en C₅-C₂₀, un aralkyle en C₇-C₂₄, un perfluoroaryle en C₅-C₂₄, ou un hétéroaryle de 5 à 12 chaînons ;
X¹ et X² désignent un ligand anionique indépendamment choisi parmi les groupes comprenant des anions halogénure, les groupes -CN, -SCN, -OR', -SR', -O(C=O)R', -O(SO₂)R' et -OSi(R')₃, où R' désigne un alkyle en C₁-C₁₂, un cycloalkyle en C₃-C₁₂, un alcényle en C₂-C₁₂, ou un aryle en C₅-C₂₀, qui peut éventuellement être substitué par au moins un alkyle en C₁-C₁₂, un perfluoroalkyle en C₁-C₁₂, un alcoxyle en C₁-C₁₂, un aryloxyle en C₅-C₂₄, un hétéroaryloxyle en
C₅-C₂₀ ou un atome d'halogène,
où le terme « perfluoroalkyle » désigne un groupe alkyle dans lequel tous les atomes d'hydrogène ont été substitués par des atomes d'halogénure identiques ou différents, et
le terme « perfluoroaryle » désigne un groupe aryle dans lequel tous les atomes d'hydrogène ont été substitués par des atomes d'halogène identiques ou différents.

2. Complexe selon la revendication 1, **caractérisé en ce qu'il est** un composé défini par la formule **1a** 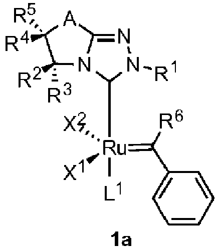 dans lequel :
R¹, R², R³, R⁴, R⁵, A, L¹, X¹ et X² ont la même définition que dans la formule 1 ;
R⁶ désigne un hydrogène.

3. Complexe selon la revendication 1, **caractérisé en ce qu'il est** un composé défini par la formule **1b** 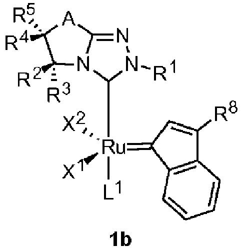 dans lequel :
R¹, R², R³, R⁴, R⁵, A, L¹, X¹ et X² ont la même définition que dans la formule 1 ;
R⁸ désigne un atome d'hydrogène, un aryle en C₅-C₂₀, un hétéroaryle en C₅-C₂₀, un aralkyle en C₇-C₂₄, vinyle ou allényle.

4. Complexe selon la revendication 1, **caractérisé en ce qu'il est** un composé défini par la formule 1c 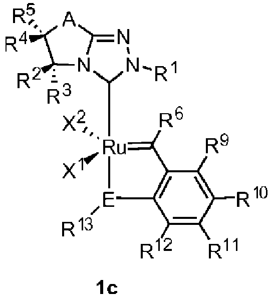 dans lequel :
R¹, R², R³, R⁴, R⁵, A, L¹, X¹ et X² ont la même définition que dans la formule **1** R⁶ désigne un hydrogène
R⁹, R¹⁰, R¹¹, R¹² indépendamment les uns des autres désignent un atome d'hydrogène, un atome d'halogène, un alkyle en C₁-C₂₅, un perfluoroalkyle en C₁-C₂₅, un alcène en C₂-C₂₅, un cycloalkyle en C₃-C₇, un alcényle en C₂-C₂₅, un cycloalcényle en C₃-C₂₅, un alcynyle en C₂-C₂₅, un cycloalcynyle en C₃-C₂₅, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons où les groupes alkyle peuvent être mutuellement reliés dans un cycle, un groupe éther (-OR'), thioéther (-SR'), nitro (-NO₂), cyanure (-CN), carboxyle (-COOH), ester (-COOR'), amide (-CONR'₂), imide (-CONR'COR'), amino (-NR'₂), amide (NR'COR'), sulfonamide (-NR'SO₂R'), sulfonyle (-SO₂R'), formyle (-CHO), sulfonamide (-SO₂NR'₂), ou cétone (-COR'), où R' a la définition suivante : un alkyle en C₁-C₅, un perfluoroalkyle en C₁-C₅, un aryle en C₅-C₂₄, un perfluoroaryle en C₅-C₂₄, un aralkyle en C₇-C₂₄, où les groupes alkyle peuvent être mutuellement reliés dans un cycle, où R⁹, R¹⁰, R¹¹, R¹² désignent de préférence un hydrogène ;
R¹³ désigne un atome d'hydrogène, un alkyle en C₁-C₂₅, un perfluoroalkyle en C₁-C₂₅, un cycloalkyle en C₃-C₇, un aryle en C₅-C₂₄, un perfluoroaryle en C₅-C₂₄, un hétéroaryle en C₅-C₂₀, un aralkyle en C₇-C₂₄, un hétérocycle de 3 à 12 chaînons où les groupes alkyle peuvent être mutuellement reliés dans un cycle, un groupe acyle -COR', cyanure (-CN), carboxyle (-COOH), ester (-COOR'), amide (-CONR'₂), sulfonyle (-SO₂R'), formyle (-CHO), sulfonamide (-SO₂NR'₂), ou cétone (-COR'), où R' a la définition suivante : un alkyle en C₁-C₅, un perfluoroalkyle en C₁-C₅, un aryle en C₅-C₂₄, un perfluoroaryle en C₅-C₂₄, un aralkyle en C₇-C₂₄ ;
E désigne un atome d'oxygène.

5. Complexe selon les revendications de 1 à 4, **caractérisé en ce que**
R¹ désigne pentafluorophényle ;
R², R³, R⁴ et R⁵ indépendamment les uns des autres désignent un atome d'hydrogène, un alkyle en C₁-C₂₅, un cycloalkyle en C₃-C₇, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons, où les groupes R², R³, R⁴ et R⁵ peuvent être mutuellement reliés dans un cycle ;
A désigne un groupe -CH₂-, -O- ou -OCH₂- ;
L¹ désigne un ligand neutre choisi parmi les groupes comprenant tricyclohexylphosphine, triphénylphosphine, pyridine, 3-bromopyridine ;
X¹ et X² désignent chlore, brome ou iode.

6. Procédé de production d'un complexe de ruthénium défini dans les revendications de 1 à 5, **caractérisé en ce que** le complexe de carbène de ruthénium défini par la formule **2** 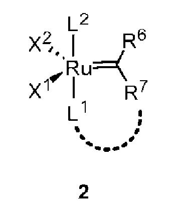 dans lequel :
R⁶ et R⁷ indépendamment l'un de l'autre désignent un atome d'hydrogène, un atome d'halogène, un alkyle en C₁-C₂₅, un perfluoroalkyle en C₁-C₂₅, un alcène en C₂-C₂₅, un cycloalkyle en C₃-C₇, un alcényle en C₂-C₂₅, un cycloalcényle en C₃-C₂₅, un alcynyle en C₂-C₂₅, un cycloalcynyle en C₃-C₂₅, un alcoxyle en C₁-C₂₅, un aryloxyle en C₅-C₂₄, un hétéroaryloxyle en C₅-C₂₀, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons où les groupes alkyle peuvent être mutuellement reliés dans un cycle, où R⁶ et R⁷ désignent de préférence un hydrogène, aryle substitué par un groupe nitro (-NO₂), cyanure (-CN), carboxyle (-COOH), ester (-COOR'), amide (-CONR'₂), sulfonyle (-SO₂R'), formyle (-CHO), sulfonamide (-SO₂NR'₂) ou cétone (-COR'), où R' a la définition suivante : un alkyle en C₁-C₅, un perfluoroalkyle en C₁-C₅, un aryle en C₅-C₂₄, un aralkyle en C₇-C₂₄, un perfluoroaryle en C₅-C₂₄,
L¹ et L² désignent un ligand neutre choisi parmi les groupes comprenant pyridine ou pyridine substituée, P(R')₃, P(OR')₃, O(R')₂, N(R')₃, où chaque R' indépendamment désigne un alkyle en C₁-C₁₂, un cycloalkyle en C₃-C₁₂, un aryle en C₅-C₂₀, un aralkyle en C₇-C₂₄, un perfluoroaryle en C₅-C₂₄, ou un hétéroaryle de 5 à 12 chaînons,
X¹ et X² désignent un ligand anionique indépendamment choisi parmi les groupes comprenant des anions halogénure, un groupe -CN, -SCN, -OR', -SR', -O(C=O)R', -O(SO₂)R' ou -OSi(R')₃, où R' désigne un alkyle en C₁-C₁₂, un cycloalkyle en C₃-C₁₂, un alcényle en C₂-C₁₂, ou un aryle en C₅-C₂₀, qui peut éventuellement être substitué par au moins un alkyle en C₁-C₁₂, un perfluoroalkyle en C₁-C₁₂, un alcoxyle en C₁-C₁₂, un aryloxyle en C₅-C₂₄, un hétéroaryloxyle en C₅-C₂₀ ou un atome d'halogène,
est soumis à une réaction avec un carbène chiral, non racémique défini par la formule 3 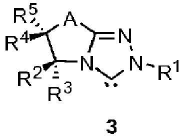 dans lequel :
R¹ désigne un perfluoroaryle en C₅-C₂₄ ;
R², R³, R⁴ et R⁵ indépendamment les uns des autres désignent un atome d'hydrogène, un atome d'halogène, un alkyle en C₁-C₂₅, un cycloalkyle en C₃-C₇, un alcoxyle en C₁-C₂₅, un aryloxyle en C₅-C₂₄, un hétéroaryloxyle en C₅-C₂₀, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons, où les groupes R², R³, R⁴ et R⁵ peuvent être mutuellement reliés dans un cycle ;
A désigne un groupe -CH₂-, -O- ou -OCH₂-.

7. Procédé de production d'un complexe de ruthénium défini dans les revendications de 1 à 5, **caractérisé en ce que** le complexe de carbène de ruthénium défini par la formule **2** 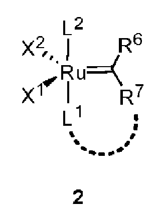 dans lequel :
R⁶ et R⁷ indépendamment l'un de l'autre désignent un atome d'hydrogène, un atome d'halogène, un alkyle en C₁-C₂₅, un perfluoroalkyle en C₁-C₂₅, un alcène en C₂-C₂₅, un cycloalkyle en C₃-C₇, un alcényle en C₂-C₂₅, un cycloalcényle en C₃-C₂₅, un alcynyle en C₂-C₂₅, un cycloalcynyle en C₃-C₂₅, un alcoxyle en C₁-C₂₅, un aryloxyle en C₅-C₂₄, un hétéroaryloxyle en C₅-C₂₀, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons où les groupes alkyle peuvent être mutuellement reliés dans un cycle, où R⁶ et R⁷ désignent de préférence un hydrogène, aryle substitué par un groupe nitro (-NO₂), cyanure (-CN), carboxyle (-COOH), ester (-COOR'), amide (-CONR'₂), sulfonyle (-SO₂R'), formyle (-CHO), sulfonamide (-SO₂NR'₂) ou cétone (-COR'), où R' a la définition suivante : un alkyle en C₁-C₅, un perfluoroalkyle en C₁-C₅, un aryle en C₅-C₂₄, un aralkyle en C₇-C₂₄, un perfluoroaryle en C₅-C₂₄,
L¹ et L² désignent un ligand neutre choisi parmi les groupes comprenant pyridine ou pyridine substituée, P(R')₃, P(OR')₃, O(R')₂, N(R')₃, où chaque R' indépendamment désigne un alkyle en C₁-C₁₂, un cycloalkyle en C₃-C₁₂, un aryle en C₅-C₂₀, un aralkyle en C₇-C₂₄, un perfluoroaryle en C₅-C₂₄, ou un hétéroaryle de 5 à 12 chaînons,
X¹ et X² désignent un ligand anionique indépendamment choisi parmi les groupes comprenant des anions halogénure, un groupe -CN, -SCN, -OR', -SR', -O(C=O)R', -O(SO₂)R' ou -OSi(R')₃ où R' désigne un alkyle en C₁-C₁₂, un cycloalkyle en C₃-C₁₂, un alcényle en C₂-C₁₂, ou un aryle en C₅-C₂₀, qui peut éventuellement être substitué par au moins un alkyle en C₁-C₁₂, un perfluoroalkyle en C₁-C₁₂, un alcoxyle en C₁-C₁₂, un aryloxyle en C₅-C₂₄, un hétéroaryloxyle en C₅-C₂₀ ou un atome d'halogène,
est soumis à une réaction avec un complexe d'argent chiral, non racémique défini par la formule **4** 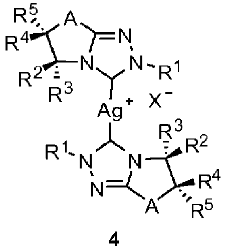 dans lequel :
R¹ désigne perfluoroaryle en C₅-C₂₄ ;
R², R³, R⁴ et R⁵ indépendamment les uns des autres désignent un atome d'hydrogène, un atome d'halogène, un alkyle en C₁-C₂₅, un cycloalkyle en C₃-C₇, un alcoxyle en C₁-C₂₅, un aryloxyle en C₅-C₂₄, un hétéroaryloxyle en C₅-C₂₀, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons, où les groupes R², R³, R⁴ et R⁵ peuvent être mutuellement reliés dans un cycle ;
A désigne un groupe -CH₂-, -O- ou -OCH₂- ;
X désigne un anion halogénure ou BF₄⁻, PF₆⁻, ou ClO₄.

8. Procédé de production d'un complexe de ruthénium défini dans les revendications 1 à 5, **caractérisé en ce que** le complexe de carbène de ruthénium défini par la formule **2** 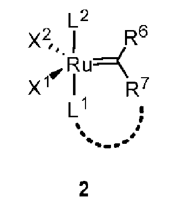 dans lequel :
R⁶ et R⁷ indépendamment l'un de l'autre désignent un atome d'hydrogène, un atome d'halogène, un alkyle en C₁-C₂₅, un perfluoroalkyle en C₁-C₂₅, un alcène en C₂-C₂₅, un cycloalkyle en C₃-C₇, un alcényle en C₂-C₂₅, un cycloalcényle en C₃-C₂₅, un alcynyle en C₂-C₂₅, un cycloalcynyle en C₃-C₂₅, un alcoxyle en C₁-C₂₅, un aryloxyle en C₅-C₂₄, un hétéroaryloxyle en C₅-C₂₀, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons où les groupes alkyle peuvent être mutuellement reliés dans un cycle, où R⁶ et R⁷ désignent de préférence un hydrogène, un aryle substitué par un groupe nitro (-NO₂), cyanure (-CN), carboxyle (-COOH), ester (-COOR'), amide (-CONR'₂), sulfonyle (-SO₂R'), formyle (-CHO), sulfonamide (-SO₂NR'₂) ou cétone (-COR'), où R' a la définition suivante : un alkyle en C₁-C₅, un perfluoroalkyle en C₁-C₅, un aryle en C₅-C₂₄, un aralkyle en C₇-C₂₄, un perfluoroaryle en C₅-C₂₄,
L¹ et L² désignent un ligand neutre choisi parmi les groupes comprenant pyridine ou pyridine substituée, P(R')₃, P(OR')₃, O(R')₂, N(R')₃, où chaque R' indépendamment désigne un alkyle en C₁-C₁₂, un cycloalkyle en C₃-C₁₂, un aryle en C₅-C₂₀, un aralkyle en C₇-C₂₄, un perfluoroaryle en C₅-C₂₄, ou un hétéroaryle de 5 à 12 chaînons,
X¹ et X² désignent un ligand anionique indépendamment choisi parmi les groupes comprenant des anions halogénure, un groupe -CN, -SCN, -OR', -SR', -O(C=O)R', -O(SO₂)R' ou -OSi(R')₃, où R' désigne alkyle en C₁-C₁₂, un cycloalkyle en C₃-C₁₂, un alcényle en C₂-C₁₂, ou un aryle en C₅-C₂₀, qui peut éventuellement être substitué par au moins un alkyle en C₁-C₁₂, un perfluoroalkyle en C₁-C₁₂, un alcoxyle en C₁-C₁₂, un aryloxyle en C₅-C₂₄, un hétéroaryloxyle en C₅-C₂₀ ou un atome d'halogène,
est soumis à une réaction avec un précurseur de carbène chiral, non racémique défini par la formule 5 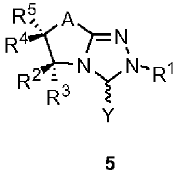 dans lequel :
R¹ désigne perfluoroaryle en C₅-C₂₄ ;
R², R³, R⁴ et R⁵ indépendamment les uns des autres désignent un atome d'hydrogène, un atome d'halogène, un alkyle en C₁-C₂₅, un cycloalkyle en C₃-C₇, un alcoxyle en C₁-C₂₅, un aryloxyle en C₅-C₂₄, un hétéroaryloxyle en C₅-C₂₀, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons, où les groupes R², R³, R⁴ et R⁵ peuvent être mutuellement reliés dans un cycle ;
A désigne un groupe -CH₂-, -O- ou -OCH₂- ;
Y désigne un alcoxyle, pentafluorophényle ou -CCl₃.

9. Procédé de production d'un complexe de ruthénium défini dans les revendications de 1 à 5, **caractérisé en ce que** le complexe de carbène de ruthénium défini par la formule 2 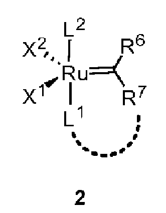 dans lequel :
R⁶ et R⁷ indépendamment l'un de l'autre désignent un atome d'hydrogène, un atome d'halogène, un alkyle en C₁-C₂₅, un perfluoroalkyle en C₁-C₂₅, un alcène en C₂-C₂₅, un cycloalkyle en C₃-C₇, un alcényle en C₂-C₂₅, un cycloalcényle en C₃-C₂₅, un alcynyle en C₂-C₂₅, un cycloalcynyle en C₃-C₂₅, un alcoxyle en C₁-C₂₅, un aryloxyle en C₅-C₂₄, un hétéroaryloxyle C₅-C₂₀, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons où les groupes alkyle peuvent être mutuellement reliés dans un cycle, où R⁶ et R⁷ désignent de préférence un hydrogène, aryle substitué par un groupe nitro (-NO₂), cyanure (-CN), carboxyle (-COOH), ester (-COOR'), amide (-CONR'₂), sulfonyle (-SO₂R'), formyle (-CHO), sulfonamide (-SO₂NR'₂) ou cétone (-COR'), où R' a la définition suivante : un alkyle en C₁-C₅, un perfluoroalkyle en C₁-C₅, un aryle en C₅-C₂₄, un aralkyle en C₇-C₂₄, un perfluoroaryle en C₅-C₂₄,
L¹ et L² désignent un ligand neutre choisi parmi les groupes comprenant pyridine ou pyridine substituée, P(R')₃, P(OR')₃, O(R')₂, N(R')₃, où chaque R' désigne indépendamment un alkyle en C₁-C₁₂, un cycloalkyle en C₃-C₁₂, un aryle en C₅-C₂₀, un aralkyle en C₇-C₂₄, un perfluoroaryle en C₅-C₂₄, ou un hétéroaryle de 5 à 12 chaînons,
X¹ et X² désignent un ligand anionique indépendamment choisi parmi les groupes comprenant des anions halogénure, un groupe -CN, -SCN, -OR', -SR', -O(C=O)R', -O(SO₂)R' ou -OSi(R')₃, où R' désigne un alkyle en C₁-C₁₂, un cycloalkyle en C₃-C₁₂, un alcényle en C₂-C₁₂, ou un aryle en C₅-C₂₀, qui peut éventuellement être substitué par au moins un alkyle en C₁-C₁₂, un perfluoroalkyle en C₁-C₁₂, un alcoxyle en C₁-C₁₂, un aryloxyle en C₅-C₂₄, un hétéroaryloxyle en C₅-C₂₀ ou un atome d'halogène,
est soumis à une réaction avec un carbène formé en conséquence de la réaction de tert-amylate de potassium ou tert-butanolate de potassium ou N,N-bis(triméthylsilyl)amide de potassium ou hydrure de sodium avec un précurseur de carbène chiral, non racémique défini par la formule **6** 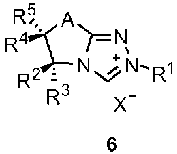 dans lequel :
R¹ désigne perfluoroaryle en C₅-C₂₄ ;
R², R³, R⁴ et R⁵ indépendamment les uns des autres désignent un atome d'hydrogène, un atome d'halogène, un alkyle en C₁-C₂₅, un cycloalkyle en C₃-C₇, un alcoxyle en C₁-C₂₅, un aryloxyle en C₅-C₂₄, un hétéroaryloxyle en C₅-C₂₀, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons, où les groupes R², R³, R⁴ et R⁵ peuvent être mutuellement reliés dans un cycle ;
A désigne un groupe -CH₂-, -O- ou -OCH₂- ;
X désigne un anion halogénure ou BF₄⁻, PF₆⁻, ou ClO₄⁻.

10. Procédé de production d'un complexe de ruthénium selon l'une quelconque des revendications de 6 à 9, **caractérisé en ce que,** en tant que complexe de carbène de ruthénium, il est utilisé un composé défini par la formule **2a** 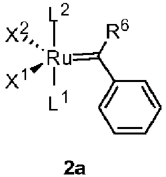 dans lequel :
X¹, X², L¹ et L² ont la même définition que dans la formule 2,
R⁶ désigne un hydrogène.

11. Procédé de production d'un complexe de ruthénium selon l'une quelconque des revendications de 6 à 9, **caractérisé en ce que,** en tant que complexe de carbène de ruthénium, il est utilisé un composé défini par la formule **2b** 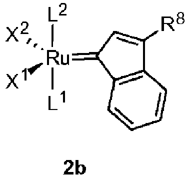 dans lequel
X¹, X², L¹ et L² ont la même définition que dans la formule 2,
R⁸ désigne un atome d'hydrogène, un aryle en C₅-C₂₀, un hétéroaryle en C₅-C₂₀, un aralkyle en C₇-C₂₄, vinyle ou allényle.

12. Procédé de production d'un complexe de ruthénium selon l'une quelconque des revendications de 6 à 9, **caractérisé en ce que,** en tant que complexe de carbène de ruthénium, il est utilisé un composé défini par la formule 2c 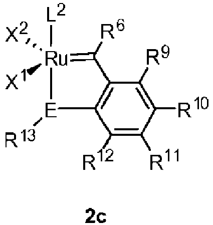 dans lequel
X¹, X², L² ont la même définition que dans la formule 2,
R⁶ désigne un hydrogène,
R⁹, R¹⁰, R¹¹, R¹² indépendamment les uns des autres désignent un atome d'hydrogène, un atome d'halogène, un alkyle en C₁-C₂₅, un perfluoroalkyle en C₁-C₂₅, un alcène en C₂-C₂₅, un cycloalkyle en C₃-C₇, un alcényle en C₂-C₂₅, un cycloalcényle en C₃-C₂₅, un alcynyle en C₂-C₂₅, un cycloalcynyle en C₃-C₂₅, un aryle en C₅-C₂₄, un hétéroaryle en C₅-C₂₀, un aralkyle en C₇-C₂₄, un perfluoroaryle en C₅-C₂₄, un hétérocycle de 3 à 12 chaînons où les groupes alkyle peuvent être mutuellement reliés dans un cycle, un groupe éther (-OR'), thioéther (-SR'), nitro (-NO₂), cyanure (-CN), carboxyle (-COOH), ester (-COOR'), amide (-CONR'₂), imide (-CONR'COR'), amino (-NR'₂), amide (NR'COR'), sulfonamide (-NR'SO₂R'), sulfonyle (-SO₂R'), formyle (-CHO), sulfonamide (-SO₂NR'₂) ou cétone (-COR'), où R' a la définition suivante : un alkyle en C₁-C₅, un perfluoroalkyle en C₁-C₅, un aryle en C₅-C₂₄, un perfluoroaryle en C₅-C₂₄, un aralkyle en C₇-C₂₄, où les groupes alkyle peuvent être mutuellement reliés dans un cycle, où R⁹, R¹⁰, R¹¹ et R¹² désignent de préférence un hydrogène ;
R¹³ désigne un atome d'hydrogène, un alkyle en C₁-C₂₅, un perfluoroalkyle en C₁-C₂₅, un cycloalkyle en C₃-C₇, un aryle en C₅-C₂₄, un perfluoroaryle en C₅-C₂₄, un hétéroaryle en C₅-C₂₀, un aralkyle en C₇-C₂₄, un hétérocycle de 3 à 12 chaînons où les groupes alkyle peuvent être mutuellement reliés dans un cycle, un groupe acyle -COR', cyanure (-CN), carboxyle (-COOH), ester (-COOR'), amide (-CONR'₂), sulfonyle (-SO₂R'), formyle (-CHO), sulfonamide (-SO₂NR'₂) ou cétone (-COR'), où R' a la définition suivante : un alkyle en C₁-C₅, un perfluoroalkyle en C₁-C₅, un aryle en C₅-C₂₄, un perfluoroaryle en C₅-C₂₄, un aralkyle en C₇-C₂₄, un perfluoroaryle en C₅-C₂₄ ;
E désigne un atome d'oxygène.

13. Procédé de production d'un complexe de ruthénium selon l'une quelconque des revendications de 6 à 12, **caractérisé en ce que** la réaction est conduite dans un solvant protique ou aprotique, un solvant chloré ou dans un solvant hydrocarbure aromatique, ou dans des mélanges de ceux-ci.

14. Utilisation d'un complexe de ruthénium défini par la formule **1** tel que défini dans les revendications de 1 à 5, en tant que (pré)catalyseur dans la métathèse et la cyclo-isomérisation d'oléfines.

15. Utilisation selon la revendication 14, **caractérisée en ce qu'**il est utilisé le complexe de ruthénium en tant que (pré)catalyseur dans la métathèse par ouverture de cycle asymétrique avec métathèse croisée (AROM/CM), métathèse croisée asymétrique (ACM), et métathèse par fermeture de cycle asymétrique (ARCM).
